# EUROPEAN PATENT SPECIFICATION

(11) **EP 1 497 321 B1**
(45) Date of publication and mention of the grant of the patent: **29.06.2011**
(21) Application number: 03711746.2
(22) Date of filing: 07.04.2003
(51) Int. Cl.: C07K 14/00, A61K 39/00, A61K 38/00, A61K 38/03

(54) **IMMUNOLOGICAL METHODS AND COMPOSITIONS FOR THE TREATMENT OF ALZHEIMER'S DISEASE**
IMMUNOLOGISCHES VERFAHREN UND ZUSAMMENSETZUNGEN FÜR DIE BEHANDLUNG DER KRANKHEIT VON ALZHEIMER
METHODES ET COMPOSITIONS IMMUNOLOGIQUES POUR LE TRAITEMENT DE LA MALADIE D'ALZHEIMER

(30) Priority: 19.04.2002 US 373914 P
(43) Date of publication of application: 19.01.2005
(60) Divisional application: 10182139.5
(73) Proprietor: THE GOVERNING COUNCIL OF THE UNIVERSITY OF TORONTO, Toronto, Ontario M5S 1A1 (CA)
(72) Inventor: ST. GEORGE-HYSLOP, Peter, Toronto, Ontario M5S 3H2 (CA); MCLAURIN, Joanne, Toronto, Ontario M5S 3H2 (CA)
(74) Representative: Adams, Harvey Vaughan John
(86) International application number: PCT/CA2003/000502
(87) International publication number: WO 2003/089460

(56) References cited:
- MONSONEGO A ET AL: "Immune hyporesponsiveness to amyloid beta-peptide in amyloid precursor protein transgenic mice: Implications for the pathogenesis and treatment of Alzheimer's disease" , PROC. NATL. ACAD. SCI, USA, VOL. 98, NR. 18, PAGE(S) 10273-10278 XP001153418 abstract page 10273, column 2, paragraph 3 page 10276, column 1 -column 2, paragraph 1 page 10277, column 1, paragraph 2 - paragraph 3 page 10277, column 2, paragraph 2 figure 5
- SPOONER E T ET AL: "The generation and characterization of potentially therapeutic Abeta antibodies in mice: differences according to strain and immunization protocol" , VACCINE, BUTTERWORTH SCIENTIFIC. GUILDFORD, GB, VOL. 21, NR. 3-4, PAGE(S) 290-297 XP004394514 ISSN: 0264-410X abstract page 292, column 1, paragraph 4 table 2 figure 2 page 295, column 1, paragraph 2 page 296, column 1, paragraph 2 -column 2, paragraph 1
- TOWN T ET AL: "CHARACTERIZATION OF MURINE IMMUNOGLOBULIN G ANTIBODIES AGAINST HUMAN AMYLOID-BETA1-42" , NEUROSCIENCE LETTERS, LIMERICK, IE, VOL. 307, NR. 2, PAGE(S) 101-104 XP001030608 ISSN: 0304-3940 abstract page 103, column 2, paragraph 2
- FRENKEL D ET AL: "N-TERMINAL EFRH SEQUENCE OF ALZHEIMER'S BETA-AMYLOID PEPTIDE REPRESENTS THE EPITOPE OF ITS ANTI-AGGREGATING ANTIBODIES" , JOURNAL OF NEUROIMMUNOLOGY, ELSEVIER SCIENCE PUBLISHERS BV, XX, VOL. 88, PAGE(S) 85-90 XP001001343 ISSN: 0165-5728

## Description

### BACKGROUND OF THE INVENTION

### FIELD OF THE INVENTION

This invention relates to immunological methods and compositions for treating Alzheimer's disease. This invention further relates to methods for identifying compounds that inhibit amyloid plaque formation and/or eliminate the existing amyloid plaques associated with Alzheimer's disease and other neuro-degenerative diseases.

### DESCRIPTION OF THE RELATED ART

Alzheimer's Disease ("AD") is a neurodegenerative brain disease that is a major cause of dementia among the elderly. Symptoms of AD can include progressive loss of learning and memory functions, personality changes, neuromuscular changes, seizures and occasionally psychotic behavior.

Alzheimer's disease is characterized by two distinct neuropathologies: the deposition of amyloid plaques in areas of the brain that are critical for memory and other cognitive functions; and the development of neurofibrillary tangles within nerve cells. It is believed that the deposition of amyloid plaques, in these critical areas of the brain, interferes with brain functions. Similarly, it has been proposed that the neurofibrillary tangles, which accumulate within nerve cells in AD patients, interfere with neuron to neuron communication.

A further characteristic of Alzheimer's disease is the presence of the hydrophobic amyloid beta peptide (Abeta₄₂) as a major constituent of amyloid plaques. The amyloid beta peptide (Abeta₄₂) is a fragment formed from proteolytic processing of a normal integral membrane protein known as amyloid protein precursor (APP) or alternatively known as Alzheimer's disease amyloid A4 protein.

Amyloid beta peptides (Abeta) comprise a group of peptides of 39-43 amino acids long that are processed from APP. See Pallitto et al., Biochemistry 38:3570-3578 (1999). The Abeta peptides generally include from 11 to 15 residues of the APP transmembrane region and therefore contain a hydrophobic region, although the entire Abeta peptide may have an amphiphillic character. See Kang et al., Nature 325:733-736 (1987). It has been shown that Abeta peptides are toxic to cells in culture. See Pike et al., Eur. J. Pharmacol. 207:367-368 (1991); Iversen et al., Biochem. J. 311:1-16 (1995). The toxicity of Abeta peptides in Alzheimer's disease is believed to be related to the process of aggregation of soluble Abeta peptides into insoluble fibrils and, subsequently, fibril incorporation into amyloid plaques. See Pike et al., Eur. J. Pharmacol. 207:367-368 (1991); Pike et al., Brain Research, 563:311-314 (1991); and Pike et al., J. Neurosci. 13:1676-1687 (1993). Similarly, Abeta peptides will form fibrils in vitro and this process can be exploited to measure inhibition of Abeta aggregation and fibril formation.

Previously, several groups have used transgenic mouse models for Alzheimer's disease wherein transgenic mice, which display both amyloid deposition in the brain and cognitive defects, were immmunized with Abeta₄₂ antigen preparations. The results from these studies demonstrated that immunization with Abeta₄₂ could produce reductions in both Alzheimer's disease-like neuropathology and the spatial memory impairments of the mice. See Schenk et al., Nature 400:173-177 (1999); Bard et al., Nature Medicine 6:916-919 (2000); Janus et al., Nature 408:979-982 (2000) and Morgan et al., Nature 408:982-982 (2000). Bard *et al* postulated that immunization with Abeta₄₂ vaccine probably leads to activation of microglia and subsequent engulfment of Abeta₄₂ aggregates by microglia. Bard et al., Nature Medicine 6:916-919 (2000). Unfortunately, all of the immunological mechanism(s) underlying the reduction in amyloid plaque deposits and improved cognitive function have not been elucidated.

Previous studies of passive administration of antibodies 3D6 and 10D5, whose epitopes are Abeta residues 1-5 and 3-6 respectively, were effective at decreasing both Abeta and amyloid plaque load in transgenic mice. See Bard et al., Nature Medicine 6:916-919 (2000). The mice were transgenic for a mutant disease-linked form of human amyloid precursor protein (APP) that was under the control of the platelet-derived (PD) growth factor promoter. These (PDAPP) mice over-express the human amyloid precursor protein and manifest many of the pathological symptoms of Alzheimer's disease. See Bard et al., Nature Medicine 6:916-919 (2000).

In another study, peripheral administration of m266, an antibody to residues 13-28 of Abeta, was shown to decrease brain Abeta burden via plasma clearance in PDAPP mice. See Demattos et al., Proc. Natl. Acad. Sci. USA 98:8850-8855 (2001). The m266 antibody is directed towards a secondary immunogenic site of Abeta, which may exhibit different binding specificity towards Abeta oligomers, protofibrils and plaques or differential access to the CNS.

Both Abeta₄₂ antigen and APP are self proteins and therefore are not normally immunogenic in an individual expressing these proteins. Consequently, attempts to produce vaccines based on these antigens necessarily require inducing autoimmunity. Moreover, any immunization protocol attempting to induce autoimmunity must carefully examine the immune responses induced by such autoantigens. In this case, it is important that any autoantigen which incorporates Abeta₄₂ or elements of Abeta₄₂ does not induce autoimmunity to the normal APP protein and disrupt its normal cellular function.

For developing effective immunotherapeutic methods for treating AD it would be desirable that the immunological mechanisms of immune mediated reduction of amyloid plaque load following immunization with Abeta₄₂ type antigens be determined.

It would be advantageous to use knowledge of the mechanism of amyloid plaque reduction to design immunogenic compositions and antigens that incorporate only those epitopes having beneficial biological activity. A further advantage is that such immunogenic compositions can be designed to exclude those epitopes inducing harmful immunity. Therefore, a need exists for defined antigens that induce very specific and limited immune responses to only aberrant forms of the Abeta antigen.

A need also exists for immunogenic compositions comprising defined antigens that can be used in immunotherapy to induce very specific and limited immune responses to only pathogenic forms of the Abeta antigen. In addition, it would be advantageous to isolate antibodies to defined Abeta epitopes having beneficial biological properties for use in passive immunotherapy. It would be further advantageous to develop diagnostic assays for determining, as soon as possible after treatment begins, whether an Alzheimer's disease patient will benefit from treatment with immunogenic compositions of Abeta antigens. A further need exists for identifying inhibitors of amyloid deposition and fibril formation.

### SUMMARY OF THE INVENTION

The present invention fulfills the foregoing needs by providing immunogenic compositions comprising residues 4-10 (SEQ ID NO:1) of the amyloid peptide Abeta₄₂ (SEQ ID NO:2) and known as Abeta(4-10), which are as defined in the appendent claims. The antigens and immunogenic compositions of the present invention are useful in treating Alzheimer's disease, for designing small molecule inhibitors of amyloid deposition and as diagnostic reagents. The disclosure further provides antibodies that bind to the Abeta₍₄₋₁₀₎ antigenic determinant. The immunogenic composition of the invention and antibodies disclosed herein can also be used in methods for' ameliorating the symptoms of Alzheimer's disease by reducing the amyloid load in Alzheimers patients.

In a first aspect, the invention provides a peptide represented by the formula:

(A)ₙ-(Th)ₘ-(B)ₒ-Abeta₍₄₋₁₀₎-(C)ₚ

wherein each of A, B and C are an amino acid residue or a sequence of amino acid residues;
wherein n, o, and p are independently integers ranging from 0 to about 20;
Th is independently a sequence of amino acid residues that comprises a helper T cell epitope;
when o is equal to 0 then Th is directly connected to the B cell epitope through a peptide bond without any spacer residues;
wherein m is an integer from 1 to about 5, and Abeta₍₄₋₁₀₎ is (SEQ ID NO: 1), wherein said peptide is selected from the group consisting of SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; and SEQ ID NO: 46.

Also disclosed herein are peptides represented by the formula

(A)ₙ -- (Th)ₘ -- (B)ₒ -- Abeta₍₄₋₁₀₎ -- (C)p

wherein each of A, B and C are an amino acid residue or a sequence of amino acid residues;
wherein n, o, and p are independently integers ranging from 0 to about 20;
Th is independently a sequence of amino acid residues that comprises a helper T cell epitope or an immune enhancing analog or segment thereof;
when o is equal to 0 then Th is directly connected to the B cell epitope through a peptide bond without any spacer residues;
wherein m is an integer from 1 to about 5 ; and'
Abeta(₄₋₁₀) is (SEQ ID NO:1), or an analog thereof containing a conservative amino acid substitution.

The present invention provides an immunogenic composition for inducing the production of antibodies that specifically bind to an amyloid-beta peptide (SEQ ID NO: 2) comprising:
(a) an antigen, comprising a T-cell epitope that provides an effective amount of T-cell help and a B-cell epitope consisting of peptide Abeta₍₄₋₁₀₎ (SEQ ID NO: 1); wherein said antigen is selected from the group consisting of SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; and SEQ ID NO: 46; and
(b) an adjuvant. > B

Also disclosed herein is an immunogenic composition for inducing antibodies which specifically bind to an amyloid-beta peptide (SEQ ID NO:2) comprising: an antigen, comprising a T-cell epitope that provides an effective amount of T-cell help and a B-cell epitope consisting of the peptide Abeta₍₄₋₁₀₎ (SEQ ID NO:1); and an adjuvant.

In a certain embodiment, the present invention provides an immunogenic composition for inducing the production of antibodies that specifically bind to an amyloid-beta peptide comprising: an antigen, comprising a T-cell epitope that provides an effective amount of T-cell help and a B-cell epitope consisting of peptide Abeta₍₄₋₁₀₎; and an adjuvant; wherein the T-cell epitope is selected from the group consisting of:
(a) one or more T-cell epitopes located N-terminal to the B-cell epitope on the same protein backbone,
(b) one or more T-cell epitopes located C-terminal to the B-cell epitope on the same protein backbone, and
(c) one or more T-cell epitopes located on a different protein backbone that is attached through a covalent linkage to the protein backbone containing the B-cell epitope.

In a particular embodiment, the present invention provides an immunogenic composition having a B-cell epitope and a T-cell epitope wherein the T-cell epitope has an amino acid sequence selected from the group consisting of SEQ ID NO:1; SEQ ID NO:2; SEQ ID NO:3; SEQ ID NO:4; SEQ ID NO:5; SEQ ID NO:6; SEQ ID NO:7; SEQ ID NO:8; SEQ ID NO:9; SEQ ID NO:10; SEQ ID NO:11; SEQ ID NO:12; SEQ ID NO:13; SEQ ID NO:14; SEQ ID NO:15; SEQ ID NO:16; SEQ ID NO:17; SEQ ID NO:18; SEQ ID NO:19; SEQ ID NO:20; and SEQ ID NO:21.

In another particular embodiment, the present invention provides an immunogenic composition comprising an antigen and an adjuvant, wherein said adjuvant comprises one or more substances selected from the group consisting of aluminum hydroxide, aluminum phosphate, saponin, Quill A, Quill A/ISCOMs, dimethyl dioctadecyl ammomium bromide/arvidine, polyanions, Freunds complete adjuvant, N-acetylmuramyl-L-alanyl-D-isoglutamine, N-acetylmuramyl-L-threonyl-D-isoglutamine, Freund's incomplete adjuvant, and liposomes.

Also disclosed herein is a method for treating an individual afflicted with Alzheimer's disease comprising administering to the individual an effective amount of an immunogenic composition for inducing the production of antibodies that specifically bind to an amyloid-beta peptide (SEQ ID NO:2) comprising: (a) an antigen, comprising a T-cell epitope that provides an effective amount of T-cell help and a B-cell epitope consisting of peptide Abeta₍₄₋₁₀₎ (SEQ ID NO:1) and (b) an adjuvant.

Also disclosed herein is a method for reducing the amount of amyloid deposits in the brain of an individual afflicted with Alzheimer's disease comprising administering to the individual an effective amount of an immunogenic composition for inducing the production of antibodies that specifically bind to an amyloid-beta peptide (SEQ ID NO:2) comprising: (a) an antigen, comprising a T-cell epitope that provides an effective amount of T-cell help and a B-cell epitope consisting of peptide Abeta₍₄₋₁₀₎ (SEQ ID NO:1) ; and (b) an adjuvant.

Also disclosed herein is a method for disaggregating the amyloid fibrils in the brain of an individual afflicted with Alzheimer's disease comprising administering to the individual an effective amount of an immunogenic composition for inducing the production of antibodies that specifically bind to an amyloid-beta peptide (SEQ ID NO:2) comprising: (a) an antigen, comprising a T-cell epitope that provides an effective amount of T-cell help and a B-cell epitope consisting of peptide Abeta₍₄₋₁₀₎ (SEQ ID NO:1); and (b) an adjuvant.

Also disclosed herein is an isolated antibody or antigen binding fragment thereof capable of binding to peptide Abeta₍₄₋₁₀₎ (SEQ ID NO:1).

Also disclosed herein is an isolated antibody or antigen binding fragment thereof capable of binding to peptide Abeta₍₄₋₁₀₎ (SEQ ID NO:1), wherein said antibody or antigen binding fragment inhibits amyloid deposition.

Also disclosed herein is an isolated antibody or antigen binding fragment thereof capable of binding to peptide Abeta₍₄₋₁₀₎ (SEQ ID NO:1), wherein said antibody or antigen binding fragment disaggregates amyloid fibrils.

Also disclosed herein is a method for treating an individual afflicted with Alzheimer's disease comprising administering to the individual an effective amount of an antibody composition which recognizes and binds to peptide Abeta₍₄₋₁₀₎ (SEQ ID NO:1).

Also disclosed herein is a method for treating an individual afflicted with Alzheimer's disease comprising administering to the individual an effective amount of an antibody composition which recognizes and: binds to peptide Abeta₍₄₋₁₀₎ (SEQ ID NO:1), wherein the antibody composition comprises polyclonal antibodies.

Also disclosed herein is a method for treating an individual afflicted with Alzheimer's disease comprising administering to the individual an effective amount of an antibody composition which recognizes and binds to peptide Abeta₍₄₋₁₀₎ (SEQ ID NO:1), wherein the antibody composition comprises a monoclonal antibody.

In still another preferred embodiment the present invention provides a method for determining if a compound is an inhibitor of amyloid deposition and fibril formation comprising: contacting the compound with the peptide Abeta₍₄₋₁₀₎ (SEQ ID NO:1); and detecting the binding of the compound with the peptide. In another embodiment, the method further comprises evaluating whether the compound inhibits amyloid fibril formation in vitro.

In another instance the present disclosure provides a diagnostic method for predicting the efficacy of an active immunization therapy for Alzheimer's disease comprising: monitoring the development of an immune response to the peptide Abeta₍₄₋₁₀₎ (SEQ ID NO:1); wherein a positive immune response to peptide Abeta₍₄₋₁₀₎ (SEQ ID NO:1) indicates that therapy should continue and a lack of immune response or a very weak immune response indicates that therapy should be discontinued.

In a further instance the present disclosure provides an immunogenic composition comprising: an antigen, and an adjuvant; wherein the antigen comprises a T-cell epitope that provides an effective amount of T-cell help and a B-cell epitope consisting of the peptide Abeta₍₄₋₁₀₎ (SEQ ID NO:1); wherein the antigen provides an effective protein structural context for inducing antibodies which bind to an immune target located in an amyloid-beta peptide (SEQ ID NO:2).

In a certain instance, the present disclosure provides an antigen, comprising a B-cell epitope, wherein the protein structural context of the B-cell epitope, which provides secondary structural mimicry of the immune target as it is found the amyloid-beta peptide (SEQ ID NO:2), is selected from the group consisting of beta-sheet, reverse turn, helix, random coil or a combination thereof. In certain further embodiments, the antigen includes a B-cell epitope comprising a mimic of the peptide Abeta₍₄₋₁₀₎ (SEQ ID NO:1).

### DETAILED DESCRIPTION OF THE INVENTION

### DEFINITIONS

The following terms, unless otherwise indicated, shall be understood to have the following meanings:
Adjuvant -- refers to substances, which can be mixtures of substances that are combined with an antigen to enhance the immunogenicity of the antigen in an immunogenic composition. Adjuvants function to increase the immune response against the antigen usually by acting directly on the immune system and by providing a slow release of the antigen.
Amyloid beta peptide (Abeta) -- refers to any one of a group of peptides of 39-43 amino acid residues that are processed from amyloid precurser protein (APP). As used herein, Abeta₄₂ refers to the 42 amino acid residue Abeta peptide. In addition, Abet₍₄₋₁₀₎ refers to the 7 amino acid residue peptide of Abeta₄₂ from residue 4 through residue 10. As discussed in more detail below, the APP gene undergoes alternative splicing to generate three common isoforms, containing 770 amino acids (APP₇₇₀), 751 amino acids (APP₇₅₁), and 695 amino acids (APP₆₉₅). By convention, the codon numbering of the longest isoform, APP₇₇₀, is used even when referring to codon positions of the shorter isoforms.
Antigen -- the antigens of the present invention are combinations of helper T-cell epitopes and B-cell epitopes. The helper T-cell epitope may be located N-terminal or C-terminal to the B-cell epitope on the same polypetide backbone. The T-cell epitope may also be located on a different polypeptide backbone that is covalently attached to the polypeptide containing the B-cell epitope, as when, for example, a small peptide is covalently linked to a carrier molecule such as keyhole limpet hemocyanin to provide immunogenicity. Alternatively, the T cell epitope may be non-covalently associated with the B-cell epitope by combining the T and B cell epitope in a composition with the adjuvant.
Antigen processing -- refers to the process where extracellular antigens from bacteria, viruses or immunogenic compositions are taken up by antigen presenting cells (APC) by endocytosis or phagocytosis. Subsequently, the antigen is fragmented by endosomes or lysosomes and peptide fragments are loaded into the binding clefts of MHC class I and MHC class II molecules. Antigen presentation -- refers to the process where MHC class I and MHC class II molecules bind short processed peptides and present these peptides on the cell surface for screening by T cells through an interaction mediated by a T cell receptor.
B-cell epitope -- refers to the part of the antigen that is the target of antibody binding and is also known as the antigenic determinant. For protein antigenic determinants, the B-cell epitope refers to amino acid residues in a particular 3-dimensional arrangement usually corresponding to the native structure. Unlike T-cell epitopes, B-cell epitopes can be exquisitely sensitive to protein conformation.
Effective amount -- refers to an amount of the immunogenic compositions, antibodies or antigen binding fragments of the invention that accomplishes any of the defined treatment goals. Effective amount is also intended to include both prophylactic and therapeutic uses of the compositions, antibodies or antigen binding fragments thereof.
Helper T-cell epitope -- helper T-cell epitopes (Th epitope) are peptides that bind to MHC class II molecules and serve to activate CD4+ T cells to provide help in the form of cytokines to B-cells for generating an antibody response to an antigen. The MHC class II molecules are loaded with processed peptide fragments of from about 7 to about 30 residues in length, in cellular compartments that communicate with the extracellular environment. Therefore, helper T cell epitopes generally represent foreign protein fragments.
Immune target -- refers to the actual 3-dimensional epitope (native) in the amyloid deposit or circulating Abeta peptides that the B-cell epitope within the antigen is attempting to mimic. Anti-protein antibodies generally are specific for particular sequences of amino acids in a particular secondary structure. Ideally, inducing antibodies to the antigen mimic of the epitope results in the production of antibodies that recognize and bind to the native epitope as it appears in the pathological amyloid deposits or circulating Abeta peptides.
Immunogen -- refers to an antigen that proves to be immunogenic.
Immunogenicity -- refers to the ability of an antigen to provoke an immune response. Antigens, in general, must be associated with antigen presenting cells in order to be immunogenic. Many factors influence immunogenicity, including antigen size, structure, sequence, degree of foreignness, presence of adjuvant, immune condition of the patient as well as other genetic factors.
Peptide -- refers to a small number, usually 2 or more, of amino acids linked together.
Polypeptide -- refers' to longer chains of amino acids linked together, but with sequence or length generally undefined. The terms protein, peptide and polypeptide' will occasionally be used interchangeably.
Promiscuous helper T cell epitope -- refers to helper T cell epitopes capable of inducing T cell activation responses (T cell help) in large numbers of individuals expressing diverse MHC haplotypes, i.e., a genetically diverse population. Such Th epitopes function in many different individuals of a heterogeneous population and' are considered to be promiscuous Th epitopes.
Protein or polypeptide backbone -- refers to the repeated unit representing an amino acid as part of a protein sequence. The polypeptide backbone consists of the sequence of three atoms: the amide nitrogen (N-H); the alpha-carbon (C); and the carbonyl carbon (C=O): which can be generally represented as follows -N-C-C-
Protein -- generally refers to specific chains of amino acids having a defined sequence, length and folded conformation, but protein, polypetide, and peptide may occasionally be used interchangebly.
Treatment or treating -- include the following goals: (1) preventing undesirable symptoms or pathological states from occurring in a subject who has not yet been diagnosed as having them; (2) inhibiting undesirable symptoms or pathological states, i.e., arresting their development; or (3) ameliorating or relieving undesirable symptoms or pathological states, i.e., causing regression of the undesirable symptoms or pathological states.

The compositions and methods of the present invention stem from the discovery by these inventors that immune mediated reductions in amyloid plaque deposits and the corresponding improvements in cognitive function can be mediated by specific antibody responses to a particular immune target or B-cell epitope in Abeta₄₂. This critical immune target was identified by the present inventors as residues 4-10 (FRHDSGY) (SEQ ID NO:1) of Abeta₄₂ which corresponds to residues 675 through 681 of the amyloid precursor protein (APP), according to the codon numbering of the longest isoform, APP₇₇₀. As a consequence, the present inventors have elucidated an important immunological mechanism of immune mediated reduction of amyloid plaque load following immunization with Abeta₄₂ type antigens.

The present inventors have discovered that antibodies recognizing and binding to residues 4-10 (FRHDSGY) (SEQ ID NO:1) of Abeta₄₂, inhibit Abeta-fibril formation and Abeta neurotoxicity. In addition, the present inventors have discovered that antibodies recognizing and binding to residues 4-10 (FRHDSGY) of Abeta₄₂, disaggregate preformed fibrils of Abeta₄₂. Further, the present invention discloses that antibodies generated during immunization with Abeta₄₂ abrogate *in vitro* cell death elicited by Abeta.

The present invention was carried out using TgCRND8 mice as a model for human AD. TgCRND8 mice are useful as a model for AD because they carry a human double mutant APP₆₉₅ transgene under the control of the prion protein promoter, and show progressive accumulation of Abeta₄₂ peptide and neuritic amyloid plaques in the cerebral cortex (a neuropathologic hallmark of AD) that is accompanied by progressive cognitive impairment. See Chishti et al., J. Biol. Chem., 276:21562-570 (2001).

The present disclosure provides antibodies specifically directed to the N-terminal peptide of Abeta that were generated during immunization of C57BL6 x C3H mice with protofibrillar forms of Abeta₄₂. The present disclosure further provides the Abeta sequence FRHDSGY (SEQ ID NO:1) corresponding to Abeta₍₄₋₁₀₎, which represents a critical epitope for protective immunity for Alzheimer's diseases. In addition, the present invention identifies the Abeta₍₄₋₁₀₎ epitope as an immune target for generating beneficial protective immunity in patients afflicted with Alzheimer's disease.

### ANTIGEN PRESENTATION

Antigen presentation refers to the molecular and cellular events where protein antigens are taken up and processed by antigen presenting cells (APC). The processed antigen fragments are then presented to effector cells, which subsequently become activated and initiate an immune response. The most active antigen presenting cells have been characterized as the macrophages (which are direct developmental products from monocytes), dendritic cells, and certain B cells.

Key molecular players in the antigen presentation and immune response.process are the MHC molecules, which are a polymorphous gene family chromosomally coded in a region known as the major histocompatibility complex *Mhc*. The MHC class I and class II molecules in humans are designated as HLA (human leucocyte antigen) molecules. Certain MHC molecules function to display unique molecular fragments on the surface of cells and to facilitate their recognition by T cells and other immune systems effector cells. See D.H. Margulies, "The Major Histocompatibility Complex", pp. 263-285 in Fundamental Immunology, Fourth Edition, Edited by W.F. Paul, Lippencott-Raven, Philadelphia, PA (1999). Further, MHC class I and class II molecules function to bind peptides in antigen-presenting cells and then to interact with αβ T cell receptors on the surface of T cells.

More specifically, MHC class I molecules bind and present samples of the cells own peptides, including endogenous, cytosolic proteins, de novo translated virus and tumor antigens. MHC class I molecules generally present peptides of from about 7 to about 16 residues in length which are recognized by CD8+ Cytotoxic T cells. MHC class I molecules are involved in effecting the cytotoxic T cell response wherein cells that are infected with a virus are killed.

The present invention is concerned primarily with T cell epitopes which serve to activate CD4+ T cells that can provide help to B-cells in generating an antibody response to an antigen. Helper T-cell epitopes (Th epitope) bind to MHC class II molecules, which are loaded with processed peptide fragments of from about 7 to about 30 residues in length, in cellular compartments that communicate with the extracellular environment. See D.H. Margulies, "The Major Histocompatibility Complex", pp. 263-285 in Fundamental Immunology, Fourth Edition, Edited by W.F. Paul, Lippencott-Raven, Philadelphia, PA (1999) (Margulies). More particularly, MHC class II molecules bind and present samples of peptides, which are ingested by the antigen presenting cell from the immediate extracellular environment, to CD4+ T cells. The CD4+ T cells then become activated and then provide help in the form of cytokines to B cells for producing antibodies. In humans, the MHC class II molecules comprise the HLA-DR, HLA-DQ and HLA-DP molecules, which occur in various genetically coded alleles.

The immunogenic compositions of the present invention comprise antigens having a B-cell epitope and a T-cell epitope that are processed and presented as protein or peptide fragments by MHC molecules on the surface of so-called "antigen-presenting cells" and are recognized by CD4+ T-lymphocytes as effector cells.

In order to assure an effective immunosurveillance, the physiology of MHC molecules is designed so that they can present as broad a spectrum of antigenic peptides as possible. Consequently, the copy number of a defined antigenic peptide on the cell surface of antigen-presenting cells is very low (magnitude 10² of a defined antigenic peptide given a total population of approximately 10⁵ peptide receptors). This means that a very heterogeneous mixture of antigenic peptides bound to MHC molecules ("peptide ligands") is exposed on the cell surface of the antigen-presenting cells.

The term "T-cell epitope" refers to a sequence of a protein which brings about an activation of CD4+ T helper (Th) lymphocytes after antigen processing and presentation of the peptide in the binding pocket of an MHC class II molecule. The alpha/beta T cell receptors on the surface of T cells interact with the peptide MHC class II complex, which serves as the stimulus for activation. As a result, the native conformation of the T cell epitope is not important, but only the primary sequence and the ability to bind to a particular MHC molecule.

The present invention relates to peptides, preferably synthetic peptides, which are capable of inducing antibodies against pathological forms of Abeta such as those found in amyloid plaques and in fibrils.

Immunogenicity of a peptide refers to the ability of the peptide to induce an antibody response comprising antibodies that specifically recognize and bind to a "B-cell epitope" or "antigenic determinant" within the peptide. See R.N. Germain, "Antigen Professing and Presentation", pp. 287-340 in Fundamental Immunology, Fourth Edition, Edited by W.F. Paul, Lippencott-Raven, Philadelphia, PA (1999) (Germain). In order to be immunogenic, a peptide containing a B-cell epitope must be presented in conjunction with an MHC class II antigen or a class II T cell epitope. The T-cell epitope is usually processed from the immunogen during antigen processing by antigen-presenting cells and then binds to the MHC class II molecule in a sequence specific manner. See Germain. This MHC class II T cell epitope complex is recognized by CD4+ T-lymphocytes (Th cells). The Th cells have the ability to cause the proliferation of specific B cells producing antibody molecules that are capable of recognizing the associated B cell epitope from the presented immunogen. Thus, the production of an antibody, which is specific for a particular B cell epitope, is linked to the presence of a T cell epitope within or associated with the immunogen.

Another complication arises when the antigen is not a foreign protein. Since Abeta is a self molecule, it should not contain any Th epitopes that induce lymphocyte activation and, thus, an antibody response against itself. Therefore, foreign T cell epitopes have to be provided by including specific sequences derived from potent foreign immunogens including tetanus toxin, pertussis toxin, the measles virus F protein and the hepatitis B virus surface antigen (HBsAg) and others. Such T cell epitope sequences may be included on the same protein backbone as the B-cell epitope, which is the Abeta₍₄₋₁₀₎ peptide. The location of the T cell epitope may be either N-terminal to the B-cell epitope or C-terminal to the B-cell epitope. Alternatively, the T cell epitope may be provided on a separate protein backbone, known as a carrier molecule, which may or may not be covalently linked to the peptide containing the B-cell epitope8.

Additional T cell epitopes can be selected by following procedures well known in the art, such as by acid elution and mass spectroscopy sequencing of MHC Class II bound peptides from immunoaffinity-purified class II molecules as disclosed in Rudensky et al., Nature 353:622-627 (1991); Chicz et al., Nature 358:764-768 (1992); and Hunt et al., Science 256:1817-1820 (1992),

Ideally, the Th epitopes selected are, preferably, capable of eliciting T cell activation responses (T cell help) in large numbers of individuals expressing diverse MHC haplotypes. This means that these epitopes function in many different individuals of a heterogeneous population and are considered to be promiscuous Th epitopes. Promiscuous Th epitopes provide an advantage of eliciting potent anti-Abeta antibody responses in most members of a genetically diverse population.

The T helper epitopes of this invention are selected not only for a capacity to cause immune responses in most members of a given population, but also for a capacity to cause memory/recall responses. The vast majority of human patients receiving Abeta immunotherapy will already have been immunized with the pediatric vaccines of measles, mumps, rubella, diphtheria, pertussis and tetanus. These patients have therefore been previously exposed to more than one of the Th epitopes present in the immunogen mixture. Such prior exposure may be useful because prior exposure to a Th epitope through immunization with the standard vaccines should establish Th cell clones, which can immediately respond and provide help for an antibody response.

The helper T-cell epitope is a sequence of amino acids (natural or non-natural amino acids) that comprises a Th epitope. A helper T-cell epitope can consist of a continuous or discontinuous epitope. Hence not every amino acid residue of a helper T-cell epitope is a required part of the epitope. Accordingly, Th epitopes, including analogs and segments of Th epitopes, are capable of enhancing or stimulating an immune response to Abeta. Immunodominant Helper T-cell epitopes are broadly reactive in animal and human populations with widely divergent MHC types. See Celis et al. J. Immunol. 140:1808-1815 (1988); Demotz et al. J. Immunol. 142:394-402 (1989); Chong et al. Infect. Immun. 60:4640-4647 (1992). The helper T-cell epitope of the subject peptides has from about 10 to about 50 amino acids, preferably from about 10 to about 40 amino acid residues, more preferably from about 10 to about 30 amino acid residues, even more preferably from about 10 to about 20 amino acid residues, or preferably from about 10 to about 15 amino acid residues. When multiple helper T-cell epitopes are present (i.e. n>2), then each helper T-cell epitope is independently the same or different.

Helper T-cell epitope may include analogs, substitutions, deletions and insertions of from one to about 10 amino acid residues in the helper T-cell epitope. The helper T-cell epitope segments are contiguous portions of a helper T-cell epitope that are sufficient to enhance or stimulate an immune response to Abeta. The helper T-cell epitope may be separated from the B-cell epitope by one or more spacer amino acid residues.

Th epitopes of the present invention include hepatitis B surface antigen helper T cell epitopes (HB-Th), pertussis toxin helper T cell epitopes (PT-Th), tetanus toxin helper T cell epitopes (TT-Th), measles virus F protein helper T cell epitopes (MV-Th), Chlamydia trachamates major outer membrane protein helper T well epitopes (CT-Th), diphtheria toxin helper T cell epitopes (DT-Th), Plasmodium falciparum circumsporozoite helper T cell epitopes (PF=Th), Schistosoma mansoni triose phosphate isomerase helper T-cell epitopes (SM-Th), Eecherichia coli Tra T helper T cell epitopes (TraT-Th) and immune-enhancing analogs and segments of any of these Th epitopes. A selection of broadly reactive Th epitopes is described in U.S. Patent No. 5,759,551 to Ladd et al., Examples of helper T cell epitope sequences are provided below:

**Table 1. Helper T-cell Epitopes**

| | |
|---|---|
| HB-Th: | |
| | Phe--Phe--Leu--Leu--Thr--Arg--Ile--Leu--thr--Ile--Pro--Gln-- Ser--Leu--Asp, SEQ ID NO:3 |
| PT-Th: | |
| | Lys--Lys--Leu--Arg--Arg--Leu--Leu--Tyr--Met--Ile--Tyr--Met-- Ser--Gly--Leu--Ala--Val--Arg--Val--His--Val--Ser--Lys--Glu-- Glu--Gln--Tyr--Tyr--Asp--Tyr, SEQ ID NO:4 |
| TT-Th: | |
| | Lys--Lys--Gln--Tyr--Ile--Lys--Ala--Asn--Ser--Lys--Phe--Ile-- Gly--Ile--Thr--Glu--Leu, SEQ ID NO:5 |
| TT2-Th: | |
| | Lys--Lys--Phe--Asn--Asn--Phe--Thr--Val--Ser--Phe--Trp--Leu-- Arg--Val--Pro--Lys--Val--Ser--Ala--Ser--His--Leu SEQ ID NO:6 |
| PT-Th: | |
| | Tyr--Met--Ser--Gly--Leu--Ala--Val--Arg--Val--His--Val--Ser-- Lys--Glu--Glu, SEQ ID NO:7 |
| TT3-Th: | |
| | Tyr--Asp--Pro--Asn--Tyr--Leu--Arg--Thr--Asp--Ser--Asp--Lys-- Asp--Arg--Phe--Leu--Gln--Thr--Met--Val--Lys--Leu--Phe--Asn-- Arg--Ile--Lys, SEQ ID NO:8 |
| PT-Th: | |
| | Gly--Ala--Tyr--Ala--Arg--Cys--Pro--Asn--Gly--Thr--Arg--Ala-- Leu--Thr--Val--Ala--Glu--Leu--Arg--Gly--Asn--Ala--Glu--Leu SEQ ID NO:9 |
| MVF1-Th: | |
| | Leu--Ser--Glu--Ile--Lys--Gly--Val--Ile--Val--His--Arg--Leu-- Glu--Gly--Val SEQ ID NO:10 |
| MVF2-Th : | |
| | Gly--Ile--Leu--Glu--Ser--Arg--Gly--Ile--Lys--Ala--Arg--Ile-- Thr--His--Val--Asp--Thr--Glu--Ser--Tyr SEQ ID NO:11 |
| TT4-Th : | |
| | Trp--Val--Arg--Asp--Ile--Ile--Asp--Asp--Phe--Thr--Asn--Glu-- Ser--Ser--Gln--Lys--Thr SEQ ID NO:12 |
| TT5-Th : | |
| | Asp--Val--Ser--Thr--Ile--Val--Pro--Tyr--Ile--Gly--Pro--Ala-- Leu--Asn--His--Val SEQ ID NO:13 |
| CT-Th : | |
| | Ala--Leu--Asn--Ile--Trp--Asp--Arg--Phe--Asp--Val--Phe--Cys-- Thr--Leu--Gly--Ala--Thr--Thr--Gly--Tyr--Leu--Lys--Gly--Asn-- Ser SEQ ID NO:14 |
| DT-Th : | |
| | Asp--Ser--Glu--Thr--Ala--Asp--Asn--Leu--Glu--Lys--Thr--Val-- Ala--Ala--Leu--Ser--Ile--Leu--Pro--Gly--His--Gly--Cys SEQ ID NO:15 |
| DT-Th : | |
| | Glu--Glu--Ile--Val--Ala--Gln--Ser--Ile--Ala--Leu--Ser--Ser-- Leu--Met--Val--Ala--Gln--Ala--Ile--Pro--Leu--Val--Gly--Glu-- Leu--Val--Asp--Ile--Gly--Phe--Ala--Ala--Thr--Asn--Phe--Val-- Glu--Ser--Cys SEQ ID NO:16 |
| PF-Th : | |
| | Asp--His--Glu--Lys--Lys--His--Ala--Lys--Met--Glu--Lys--Ala-- Ser--Ser--Val--Phe--Asn--Val--Val--Asn--Ser SEQ ID NO: 17 |
| SM-Th : | |
| | Lys--Trp--Phe--Lys--Thr--Asn--Ala--Pro--Asn--Gly--Val--Asp-- Glu--Lys--His--Arg--His SEQ ID NO:18 |
| TraT1-Th : | |
| | Gly--Leu--Gln--Gly--Lys--Hfis--Ala--Asp--Ala--Val--Lys--Ala- Lys--Gly SEQ ID NO:19 |
| TraT2-Th : | |
| | Gly--Leu--Ala--Ala--Gly--Leu--Val--Gly--Met--Ala--Ala--Asp-- Ala--Met--Val--Glu--Asp--Val--Asn SEQ ID NO:20 |
| TraT-Th : | |
| | Ser--Thr--Glu--Thr--Gly--Asn--Gln--His--His--Tyr--Gln--Thr-- Arg--Val--Val--Ser--Asn--Ala--Asn--Lys SEQ ID NO:21 |

In certain embodiments, the present invention has a T-cell epitope having an amino acid sequence selected from the group consisting of SEQ ID NO:3; SEQ ID NO:4; SEQ ID NO:5; SEQ ID NO:6; SEQ ID NO:7; SEQ ID NO:8; SEQ ID NO:9; SEQ ID NO:10; SEQ ID NO:11; SEQ ID NO:12; SEQ ID NO:13; SEQ ID NO:14; SEQ ID NO:15; SEQ ID NO:16; SEQ ID NO:17; SEQ ID NO:18; SEQ ID NO:19; SEQ ID NO:20; and SEQ ID NO:21.

### ANTIGEN DESIGN

The immunogenic compositions of the present invention include an antigen, comprising a T-cell epitope that provides an effective amount of T-cell help and a B-cell epitope consisting of the peptide Abeta₍₄₋₁₀₎

The antigen peptides of this disclosure are represented by the following formulas:

I. (A)ₙ (Th)ₘ (B)o -- Abeta₍₄₋₁₀₎ -- (C)ₚ

II. (A)ₙ -- Abeta₍₄₋₁₀₎ -- (B)ₒ -- (Th)ₘ -- (C)p

III. (D)_{q} -- Abeta₍₄₋₁₀₎ -- (E)r

wherein A, C, D, and E are independently an amino acid residue or a sequence of amino acid residues;
wherein B, a spacer, is an amino acid residue or a sequence of amino acid residues; when o is equal to 0 then the Th is directly connected to the B cell epitope through a peptide bond without any spacer residues;
wherein n, o, and p are independently integers ranging from 0 to about 20; when o is equal to 0 then the Th is directly connected to the B cell epitope without any spacer residues;
wherein m is an integer from 1 to about 5;
wherein q and r are independently integers ranging from 0 to about 100;
Th is independently a sequence of amino acid residues that comprises a helper T cell epitope or an immune enhancing analog or segment thereof; or an analog thereof containing a conservative amino acid substitution; Th may be tandomly repeated;
Abeta₍₄₋₁₀₎ is residues 4-10 (FRHDSGY) of Abeta₄₂ SEQ ID NO:1, or an analog thereof containing a conservative amino acid substitution; Abeta₍₄₋₁₀₎ SEQ ID NO:1 may be tandomly repeated or otherwise present in multiple copies.

The invention also includes in so far as these are encompassed by the appendent claims, compositions, of two or more of the peptides represented by formulas I, II and III. One or more peptides of Formula I can be combined to form compositions. Alternatively, one or more peptides from formulas I, II, and III may be combined to form mixtures or compositions.

The antigen peptides of the present disclosure have from about 20 to about 100 amino acid residues, alternatively from about 20 to about 80 amino acid residues. In a certain embodiment, the antigen peptides of the present invention have from about 20 to about 60 amino acid residues, preferably from about 20 to about 50 amino acid residues, and more preferably has from about 25 to about 40 amino acid residues. In another preferred embodiment, the antigen peptide has from about 20 to about 35 amino acid residues.

When A, B, C, D and E are amino acid residues, then they can be any non-naturally occurring amino acid or any naturally occurring amino acid. Non-naturally occurring amino acids include, but are not limited to, beta-alanine, ornithine, norleucine, norvaline, hydroxyproline, thyroxine, gamma-amino butyric acid, homoserine, citrulline and the like. Naturally-occurring amino acids include alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine and valine. Moreover, when m is at least one, and two or more of the A, B, C, D or E groups are amino acids, then each amino acid is independently the same or different.

The amino acids of A, B, C, D or E groups may be modified with fatty acids. For example, 1 or more epsilon-palmitoyllysines may be added N-terminal and C-terminal to the Abeta epitope and the entire peptide can be anchored onto the surface of vesicles. The vesicles may contain the immunostimulator lipid A. See Nicolau et al., Proc. Natl. Acad. Sci. USA 99:2332-2337 (2002). .

The Abeta₍₄₋₁₀₎ epitope may be incorporated into protein dendrimers through the use of an orthogonal coupling strategy for construction of protein antigens. Specially constructed dendrimers may form the basis for the assembly of effective vaccine antigens, including, for example, a multiple antigen peptide constriction as described in US Patent No.6,310,810 to Tam.

### SYNTHETIC PEPTIDES AS ANTIGENS AND VACCINES

In many cases, the use of an entire protein or glycoprotein as an immunogen for the development of effective vaccines and immunotherapies for human diseases and infectious agents has proven either ineffective due to a lack of immunogenicity, or results in the enhancement of infection and disease due to the inclusion of nonprotective epitopes. See Osterhaus et al, Vaccine, 7:137-141 (1989) ; Gilbert et al. Virus Research, 7:49-67 (1987); Burke, D. Perspect. Biol. Med., 35:511-530 (1992).

The use of synthetic peptide antigens in vaccines or in immunogenic compositions can circumvent many of the problems associated with recombinant vaccines. The advantages of using synthetic peptides that correspond to specific protein domains include: selection and inclusion of only protective epitopes; exclusion of disease enhancing epitopes; exclusion of harmful autoimmune epitopes; exclusion of infectious material; and, synthetic peptides antigens are chemically well defined and can be produced at a reasonable cost. See Arnon and Horwitz, Curr. Opin. Immunol., 4:449-453, (1992).

The disadvantages are that small synthetic peptides may not contain the precise amino acid sequences necessary for processing and binding to major histocompatibility complex (MHC) class I and class II proteins, for presentation to the immune system. See Rothbard, Biotechnology, 20:451-465, (1992). Another disadvantage is that the 3-dimensional solution structure of small peptides may be different than that found in the native protein and, therefore, the peptide may not induce humoral immunity of the proper specificity and affinity to provide protective immunity. See Bernard et al. Aids Res. and Hum. Retroviruses, 6:243-249, (1990).

The peptide antigens of the present invention can be prepared in a wide variety of ways. The peptide, because of its relatively small size, can be synthesized in solution or on a solid support in accordance with conventional techniques. Various automatic and manual synthesizers are commercially available today and can be used in accordance with known protocols. See, for example, US Patent No.5,827,666 to Finn et al.; Stewart and Young, Solid Phase Peptide Synthesis, 2nd ed., Pierce Chemical Co., 1984; and Tam et al., J. Am Chem. Soc. (1983) 105:6442

Alternatively, hybrid DNA technology can be employed where a synthetic gene is prepared by employing single strands which code for the polypeptide or substantially complementary strands thereof, where the single strands overlap and can be brought together in an annealing medium so as to hybridize. The hybridized strands then can be ligated to form the complete gene, and, by choice of appropriate termini, the gene can be inserted into an expression vector, many of which are readily available today. See, for example, e.g., Sambrook, Fritsch & Maniatis, Molecular Cloning: A Laboratory Manual, Second Edition (1989) Cold Spring Harbor Laboratory Press, Cold Spring Harbor, N.Y. (herein "Sambrook, et al., 1989"); and expressed in procaryotic or eukaryotic expression systems to produce the desired peptides.

### CARRIERS

The Abeta₍₄₋₁₀₎ epitope antigens of the invention, such as described within this application may be conjugated to a carrier molecule to provide T cell help.

Carrier molecules to which antigens of the invention are covalently linked (conjugated) are advantageously, non-toxic, pharmaceutically acceptable and of a size sufficient to produce an immune response in mammals. Examples of suitable carrier molecules include tetanus toxoid, keyhole limpet hemocyanin (KLH), and peptides corresponding to T cell epitopes (that is, T1 and T2) of the gp120 envelope glycoprotein that can substitute for non-AIDS virus-derived carrier molecules (Cease, Proc. Nat '1. Acad. Sci. (USA) 84:4249, 1987; Kennedy et al., J. Biol. Chem. 262:5769, 1987). Peptides can also be administered with a pharmaceutically acceptable adjuvant, for example, alum, or conjugated to other carrier molecules more immunogenic than tetanus toxoid.

Linkage of a carrier molecule to a peptide antigen of the invention can be direct or through a spacer molecule. Spacer molecules are, advantageously, nontoxic and reactive. Two glycine residues added to the amino terminal end of the peptide can provide a suitable spacer molecule for linking Abeta₍₄₋₁₀₎ sequences, or portions thereof, to a carrier molecule; alternatively, Abeta₍₄₋₁₀₎ sequences, or portions thereof, can for example be synthesized directly adjacent to, for example, another immunogenic amyloid sequence. Cysteines can be added either at the N or C terminus of the Abeta₍₄₋₁₀₎ peptide for conjugation to the carrier molecule or to both ends to facilitate interchain polymerization via di-sulfide bond formation to form larger molecular aggregates. Conjugation of the carrier molecule to the peptide is accomplished using a coupling agent. Advantageously, the heterofunctional coupling agent M-maleimidobenzoyl-N-hydroxysuccinimide ester (MBS) or the water soluble compound m-maleimidobenzoylsulfosuccinimide ester (sulfo-MBS) is used, as described by Green et al., Cell, 28:477 (1982) ; and by Palker et al., Proc. Nat'1 Acad. Sci. U.S.A 84:2479 (1987). Many other coupling agents, such as glutaraldehyde, are available for coupling peptides to other molecules. Conjugation methods are well known in the art. See for example chapter 9 (pages 419-455) and chapter 11 (pages 494-527) of Bioconjugate Techniques by G.T. Hermanson, Academic Press, San Diego 1996).

### ADJUVANTS

Two of the characteristic features of antigens are their immunogenicity or their capacity to induce an immune response *in vivo* (including the formation of specific antibodies), and their antigenicity, that is, their capacity to be selectively recognized by the antibodies that are specific for that sequence and structure.

Some antigens are only weakly immunogenic when administered by itself. Consequently, a weakly immunogenic antigen may fail to induce the immune response necessary for providing effective immunotherapy or protection for the organism.

The immunogenicity of an antigen can be increased by administering it as a mixture with additional substances, called adjuvants. Adjuvants function to increase the immune response against the antigen either by acting directly on the immune system and by providing a slow release of the antigen. Thus, the adjuvant modifies the pharmacokinetic characteristics of the antigen and increases the interaction time between the antigen with the immune system. The use of adjuvants is well known in the art and many suitable adjuvants can be used. The preparation of immunogenic compositions and the use of adjuvants is generally described in vaccine Design--The subunit and adjuvant approach (Ed. Powell and Newman) Pharmaceutical Biotechnology Vol. 6 Plenum Press 1995.

The most widespread adjuvants are Freund's adjuvant, an emulsion comprising dead mycobacteria in a saline solution within mineral oil and Freund's incomplete adjuvant, which does not contain mycobacteria.

Adjuvants are capable of either increasing the intensity of the immune response to the antigen or of producing a specific activation of the immune system. There are five general categories of adjuvant including (1) aluminum salts, such as aluminum hydroxide or aluminum phosphate (2) surface active agents, such as saponin and Quill A, Quill A/ISCOMs, dimethyl dioctadecyl ammomium bromide/arvidine (3) polyanions, (4) bacterial derivatives, such as Freunds complete, N-acetylmuramyl-L-alanyl-D-isoglutamine (muramyl dipeptides), N-acetylmuramyl-L-threonyl-D-isoglutamine (threonyl MDP) (5) vehicles and slow release materials, such as Freund's incomplete (oil emulsion), liposomes. See New Generation Vaccines, Chapter 11, pages 129-140, Adjuvants for a New Generation of Vaccines by A.C. Allison and N.E. Byars, Marcel Dekker, New York, 1990).

The immunogenic compositions of the present invention comprise an antigen and an adjuvant. Suitable adjuvants include alum, which is an aluminum salt such as aluminum hydroxide gel or aluminum phosphate, but may also be a salt of calcium, iron or zinc. Other suitable adjuvants include insoluble suspensions of acylated tyrosine, or acylated sugars, cationically or anionically derivatised polysaccharides, or polyphosphazenes.

Combinations of adjuvants may be used to create an adjuvant system. Suitable adjuvant systems include, for example, a combination of monophosphoryl lipid A, preferably 3-de-o-acylated monophosphoryl lipid A (3D-MPL) together with an aluminum salt. An alternative adjuvant system comprises, for example the RIBI ADJUVANT SYSTEM™, which is a combination of monophosphoxyl lipid A, preferably 3-de-o-acylated monophosphoryl lipid A (3D-MPL), synthetic trehalose dicorynomycolate and cell wall skeleton materials. An enhanced system involves the combination of a monophosphoryl lipid A and a saponin derivative particularly the combination of QS21 and 3D-MPL as disclosed in WO 94/00153, or a less reactogenic composition where the QS21 is quenched with cholesterol as disclosed in WO 96/33739. A particularly potent adjuvant formulation involving QS21, 3D-MPL & tocopherol in an oil in water emulsion is described in WO 95/17210 and is a preferred formulation. The disclosures of WO 94/00153, WO 96/33739 and WO 95/17210 .

Alternatively, the immunogenic compositions of the present invention may be encapsulated.within liposomes or vesicles as described by Fullerton, U.S. Pat. No. 4,235,877.

### ANTIBODY STRUCTURE

The present disclosure contemplates antibodies or antigen binding fragments thereof, which bind to the Abeta₍₄₋₁₀₎ epitope and inhibit amyloid deposition and fibril formation. In general, the basic antibody structural unit is known to comprise a tetramer. Each tetramer includes two identical pairs of polypeptide chains, each pair having one "light" (about 25 kDa) and one "heavy" chain (about 50-70 kDa). The amino-terininal portion of each chain may include a variable region of about 100 to 110 or more amino acids primarily responsible for antigen recognition. The carboxy-terminal portion of each chain may define a constant region primarily responsible for effector function. Typically, human light chains are classified as kappa and lambda light chains. Furthermore, human heavy chains are typically classified as mu, delta, gamma, alpha, or epsilon, and define the antibody's isotype as IgM, IgD, IgG, IgA, and IgE, respectively. Within light and heavy chains, the variable and constant regions are joined by a "J" region of about 12 or more amino acids, with the heavy chain also including a "D" region of about 10 more amino acids. See J.K. Frazer and J.D. Capra, "Immunoglobulins: Structure and Function", pp. 37-75 in Fundamental Immunology, Fourth Edition, Edited by W.F. Paul, Lippencott-Raven, Philadelphia, PA (1999) (Frazer).

The variable regions of each light/heavy chain pair may form the antibody binding site. Thus, in general, an intact IgG antibody has two binding sites. Except in bifunctional or bispecific antibodies, the two binding sites are, in general, the same.

Normally, the chains all exhibit the same general structure of relatively conserved framework regions (FR) joined by three hypervariable regions, also called complementarity determining regions or CDRs. The CDRs from the two chains of each pair are usually aligned by the framework regions, enabling binding to a specific epitope. In general, from N-terminal to C-terminal, both light and heavy chains comprise the domains FR1, CDR1, FR2, CDR2, FR3, CDR3 and FR4. The assignment of amino acids to each domain is, generally, in accordance with the definitions of Kabat Sequences of Proteins of Immunological Interest (National Institutes of Health, Bethesda, Md. (1987 and 1991)), or Chothia, et al., J Mol. Biol. 196:901-917 (1987); Chothia, et al., Nature 342:878-883 (1989).

### TYPES OF ANTIBODY

The term "antibody molecule" includes, but is not limited to, antibodies and fragments thereof . The term includes monoclonal antibodies, polyclonal antibodies, bispecific antibodies, Fab antibody fragments, F(ab)₂ antibody fragments, Fv antibody fragments (*e.g*., V_{H} or V_{L}), single chain Fv antibody fragments and dsFv antibody fragments. Furthermore, the antibody molecules may be fully human antibodies, humanized antibodies or chimeric antibodies. Preferably, the antibody molecules are monoclonal, fully human antibodies.

The anti-Abeta₍₄₋₁₀₎ antibody molecules preferably recognize human amyloid Abeta proteins and peptides; however, the present disclosure includes antibody molecules which recognize amyloid Abeta proteins and peptides from different species, preferably mammals (*e.g*., mouse, rat, rabbit, sheep or dog).

In addition, anti-Abeta₍₄₋₁₀₎ antibody may be derived from a human monoclonal antibody. Such antibodies are obtained from transgenic mice that have been "engineered" to produce specific human antibodies in response to antigenic challenge. In this technique, elements of the human heavy and light chain locus are introduced into strains of mice derived from embryonic stem cell lines that contain targeted disruptions of the endogenous heavy chain and light chain loci. The transgenic mice can synthesize human antibodies specific for human antigens, and the mice can be used to produce human antibody-secreting hybridomas. Methods for obtaining human antibodies from transgenic mice are described by Green et al., Nature Genet. 7:13 (1994), Lonberg et al., Nature 368:856 (1994), and Taylor et al., Int. Immun. 6:579 (1994).

In a preferred embodiment, fully-human monoclonal antibodies directed against Abeta₍₄₋₁₀₎ are generated using transgenic mice carrying parts of the human immune system rather than the mouse system. These transgenic mice, which may be referred to, herein, as "HuMAb" mice, contain a human immunoglobulin gene miniloci that encodes unrearranged human heavy (mu and gamma) and kappa light chain immunoglobulin sequences, together with targeted mutations that inactivate the endogenous mu and kappa chain loci (Lonberg, N., et al., (1994) Nature 368 (6474) : 856-859). Accordingly, the mice exhibit reduced expression of mouse IgM or kappa, and in response to immunization, the introduced human heavy and light chain transgenes undergo class switching and somatic mutation to generate high affinity human IgG monoclonal antibodies (Lonberg, N., *et al*., (1994), supra; reviewed in Lonberg, N. (1994) Handbook of Experimental Pharmacology 113:49-101; and Lonberg, N., et al., (1995) Intern. Rev. Immunol. 13:65-93. The preparation of HuMab mice is commonly known in the art and is described, for example, in Lonberg, et al., (1994) Nature 368(6474): 856-859; Lonberg, N. (1994) Handbook of Experimental Pharmacology 113:49-101; Lonberg, N., et al., (1995) Intern. Rev. Immunol. Vol. 13 : 65-93; Fishwild, D., et al., (1996) Nature Biotechnology 14 : 845-851. See further, U.S. Patent Nos. 5,814,318; 5,874,299; and 5,770,429; all to Lonberg and Kay, and GenPharm International; U.S. Patent No. 5,545,807 to Surani, et al.,

To generate fully human monoclonal antibodies to Abeta₍₄₋₁₀₎, HuMab mice can be immunized with an immunogenic composition comprising the Abeta₍₁₋₁₀₎ antigen of the present invention. Preferably, the mice will be 6-16 weeks of age upon the first immunization. For example, an immunogenic composition comprising the Abeta₍₄₋₁₀₎ antigen of the present invention can be used to immunized the HuMab mice intraperitoneally. The mice can also be immunized with whole HEK293 cells that are stably transformed or transfected with an Abeta₍₄₋₁₀₎ containing gene. An "antigenic Abeta₍₄₋₁₀₎ polypeptide" may refer to an Abeta₍₄₋₁₀₎ polypeptide of any fragment thereof which elicits an anti-Abeta₍₄₋₁₀₎ immune response in HuMab mice.

In general, HuMAb transgenic mice respond best when initially immunized intraperitoneally (IP) with antigen in complete Freund's adjuvant, followed by every other week IP immunizations (usually, up to a total of 6) with antigen in incomplete Freund's adjuvant. Mice can be immunized, first, with cells expressing Abeta₍₄₋₁₀₎ (*e.g*., stably transformed HEK293 cells), then with a soluble fragment of an antigen containing Abeta₍₄₋₁₀₎ such as the immunogenic-compositions of the present invention, and continually receive alternating immunizations with the two antigens. The immune response can be monitored over the course of the immunization protocol with plasma samples being obtained by retro-orbital or tail bleeds. The plasma can be screened for the presence of anti-Abeta₍₄₋₁₀₎ antibodies, for example by ELISA, and mice with sufficient titers of immunoglobulin can be used for fusions. Mice can be boosted intravenously with antigen 3 days before sacrifice and removal of the spleen. It is expected that 2-3 fusions for each antigen may need to be' performed. Several mice can be immunized for each antigen. For example, a total of twelve HuMAb mice of the HC07 and HC012 strains can be immunized.

Hybridoma cells that produce the monoclonal, fully human anti-Abeta₍₄₋₁₀₎ antibodies may then be produced by methods that are commonly known in the art. These methods include, but are not limited to, the hybridoma technique originally developed by Kohler, et al., Nature 256:495-497 (1975); as well as the trioma technique Hering, et al., Biomed. Biochim. Acta. 47:211-216 (1988) and Hagiwara, et al., Hum. Antibod. Hybridomas 4:15 (1993); the human B-cell hybridoma technique (Kozbor, et al., Immunology Today 4:72 (1983); and Cote, et al., Proc. Natl . Acad. Sci . U.S.A 80:2026-2030 (1983) ; and the EBV-hybridoma technique (Cole, et al., in Monoclonal Antibodies and Cancer Therapy, Alan R. Liss, Inc., pp. 77-96, 1985). Preferably, mouse splenocytes are isolated and fused with PEG to a mouse myeloma cell line based upon standard protocols. The resulting hybridomas are then screened for the production of antigen-specific antibodies. For example, single cell suspensions of splenic lymphocytes from immunized mice are fused to one-sixth the number of P3X63-Ag8.653 nonsecreting mouse myeloma cells (ATCC, CRL 1580) with 50% PEG. Cells are plated at approximately 2 x 10⁵ cells in flat bottom microtiter plate, followed by a two week incubation in selective medium containing 20% fetal Calf Serum, 18% "653" conditioned media, 5% origen (IGEN), 4 mM L-glutamine, 1 mM L-glutamine, 1 mM sodium pyruvate, 5mM HEPES, 0.055 mM 2-mercaptoethanol, 50 units/ml penicillin, 50 mg/ml streptomycin, 50 mg/ml gentamycin and 1X HAT (Sigma; the HAT is added 24 hours after the fusion). After two weeks, cells are cultured in medium in which the HAT is replaced with HT. Individual wells are then screened by ELISA for human anti-Abeta₍₄₋₁₀₎ monoclonal IgM and IgG antibodies. Once extensive hybridoma growth occurs, medium is observed usually after 10-14 days. The antibody secreting hybridomas are replated, screened again, and if still positive for human IgG, anti-Abeta₍₄₋₁₀₎ monoclonal antibodies, can bye subcloned at least twice by limiting dilution. The stable subclones are then cultured in vitro to generate small amounts of antibody in tissue culture medium for characterization.

The anti-Abeta antibody molecules may also be produced recombinantly (*e.g*., in an *E.coli*/T7 expression system as discussed above). In this embodiment, nucleic acids encoding the antibody molecules (*e.g*., V_{H} or V_{L}) may be inserted into a pet-based plasmid and expressed in the *E.coli*/T7 system. There are several methods to produce recombinant antibodies that are well known in the art. One example of a method for recombinant production of antibodies is disclosed in U.S. Patent No. 4,816,567. The antibody molecules may also be produced recombinantly in CHO or NSO cells.

The term "monoclonal antibody," as used herein, refers to an antibody obtained from a population of substantially homogeneous antibodies, i.e., the' individual antibodies comprising the population are identical except for possible naturally occurring mutations that may be present in minor amounts. Monoclonal antibodies are highly specific, being directed against a single antigenic site. Monoclonal antibodies are advantageous in that they may be synthesized by a hybridoma culture, essentially uncontaminated by other immunoglobulins. The modifier "monoclonal" indicates the character of the antibody as being obtained from a substantially homogeneous population of antibodies, and is not to be construed as requiring production of the antibody by any particular method. As mentioned above, the monoclonal antibodies to be used in accordance with the present invention may be made by the hybridoma method first described by Kohler, et al., Nature 256:495 (1975).

A polyclonal antibody is an antibody, which was produced among or in the presence of one or more other, non-identical antibodies. In general, polyclonal antibodies are produced from a B-lymphocyte in the presence of several other B-lymphocytes, which produced non-identical antibodies. Usually, polyclonal antibodies are obtained directly from an immunized animal.

The term "fully human antibody" refers to an antibody, which comprises human immunoglobulin sequences only. Similarly, "mouse antibody refers to an antibody which comprises mouse immunoglobulin sequences only.

The present disclosure includes "chimeric antibodies"- an antibody which comprises variable region of the present disclosure fused or chimerized with an antibody region (*e.g*., constant region) from another, non-human species (*e.g*., mouse, horse, rabbit, dog, cow, chicken). These antibodies may be used to modulate the expression or activity of Abeta₍₄₋₁₀₎ in the non-human species.

"Humanized antibody" refers to an antibody which includes a non-human CDR within the framework of an otherwise human antibody or a non-human variable region attached to the constant region of an otherwise human antibody. The present disclosure contemplates humanized antibodies, which include a CDR or variable region from a non-human species, which comprises the amino acid sequence of a variable region or CDR of the present disclosure

Depending on the amino acid sequences of the constant domain of their heavy chains, immunoglobulins can be assigned to different classes. There are at least five major classes of immunoglobulins: IgA, IgD, IgE, IgG and IgM, and several of these may be further divided into subclasses (isotypes), *e.g*. IgG-1, IgG-2, IgG-3 and IgG-4; TgA-1 and IgA-2. Preferably, the antibody molecules of the disclosure are IgG-1 or IgG-4.

The antibodies of the disclosure may also be conjugated with radioisotopic labels such as ⁹⁹Tc, ⁹⁰Y, ¹¹¹In, ³²P, ¹⁴C, ¹²⁵I, ³H, ¹³¹I, ¹¹C, ¹⁵O, ¹³N, ¹⁸F, ³⁵S, ⁵¹Cr, ⁵⁷To, ²²⁶Ra, ⁶⁰Co, ⁵⁹Fe, ⁵⁷Se, ¹⁵²Eu, ⁶⁷CU, ²¹⁷Ci, ²¹¹At, ²¹²Pb, ⁴⁷Sc, ¹⁰⁹Pd, ²³⁴Th, and ⁴⁰K and non-radioisotopic labels such as ¹⁵⁷Gd, ⁵⁵Mn, ⁵²Tr, ⁵⁶Fe.

The antibodies of the disclosure may also be conjugated with fluorescent or chemilluminescent labels, including fluorophores such as rare earth chelates, fluorescein and its derivatives, rhodamine and its derivatives, isothiocyanate, phycoerythrin, phycocyanin, allophycocyanin, o-phthaladehyde, fluorescamine, ¹⁵²Eu, dansyl, umbelliferone, luciferin, luminal label, isoluminal label, an aromatic acridinium ester label, an imidazole label, an acridimium salt label, an oxalate ester label, an aequorin label, 2,3-dihydiophthalazinediones, biotin/avidin, spin labels and stable free radicals.

Any method known in the art for conjugating the antibody molecules of the disclosure to the various moieties may be employed, including those methods described by Hunter, et al., Nature 144:945 (1962) ; David, et al., Biochemistry 13:1014 (1974) ; Pain, et al., J. Immunol. Meth. 40:219 (1981); and Nygren, J., Histochem. and Cytochem. 30:407 (1982). Methods for conjugating antibodies are conventional and very well known in the art.

The present invention also relates to certain therapeutic methods based upon administration of immunogenic compositions comprising Abeta₍₄₋₁₀₎ or molecules that bind to Abeta peptides. Thus, antigens comprising Abeta₍₄₋₁₀₎ may be administered to inhibit or potentate plaque deposition in aging, or human diseases such as Alzheimer's disease.

The present invention also relates to methods of making, identifying, purifying, characterizing Abeta₍₄₋₁₀₎ antigens and analogs thereof; and methods of using Abeta₍₄₋₁₀₎ antigens and analogs thereof. Abeta₍₄₋₁₀₎ antigens can be produced by modifications including proteolytic cleavage of larger amyloid peptides isolated from natural sources, through genetic engineering techniques, or chemical synthesis, e.g., by solid phase peptide synthesis; or produced de novo by genetic engineering methodology or solid phase peptide synthesis.

### MOLEULAR BIOLOGY

In accordance with the present invention there may be employed conventional molecular biology, microbiology, and recombinant DNA techniques within the skill of the art. Such techniques are explained fully in the literature. See, e.g., Sambrook, Fritsch & Maniatis, Molecular Cloning: A Laboratory Manual, Second Edition (1989) Cold Spring Harbor Laboratory Press, Cold Spring Harbor, N.Y. (herein "Sambrook et al., 1989"); DNA Cloning: A Practical Approach, Volumes I and II (D. N. Glover ed. 1985) ; oligonucleotide Synthesis (M. J. Gait ed. 1984); Nucleic Acid Hybridization [B. D. Hames & S. J. Higgins eds. (1985)]; Transcription And Translation [B. D. Hames & S. J. Higgins, eds. (1984)]; Animal Cell Culture [R. I. Freshney, ed. (1986)]; Immobilized Cells And Enzymes [IRL Press, (1986)]; B. Perbal, A Practical Guide To Molecular Cloning (1984) ; F. M. Ausubel et al. (eds.), Current Protocols in Molecular Biology, John Wiley & Sons, Inc. (1994).

### CRND8 Mice

TgCRND8 Mice are an animal model of AD that exhibit high levels of Abeta synthesis and amyloid deposition in the CNS by 3 months of age. See International Publication No. WO01/97607 published 12/27/01. Furthermore, TgCRND8 mice exhibit cognitive changes within the time period in which amyloid deposition commences. The transgenic TgCRND8 mouse model is characterized by a great similarity to the naturally occurring Alzheimer's Disease phenotype, based on the expression of Abeta amyloid protein in the CNS, as well as on histological analysis, necrology and behavioural deficits.

The APP gene undergoes alternative splicing to generate three common isoforms. The longest isoform, containing 770 amino acids (APP₇₇₀), and the second longest isoform containing 751 amino acids (APP₇₅₁), are expressed in most tissues. The third transcript, which contains 695 amino acids (APP₆₉₅), is predominantly expressed in the brain. By convention, the codon numbering of the longest isoform, APP₇₇₀, is used even when referring to codon positions of the shorter isoforms.

The TgCRND8 transgenic mouse contains a transgene expressing a mutant form of the brain-specific APP₆₉₅ isoform; this transgene carries both the "Swedish" and "Indiana" APP mutations.

An APP₆₉₅ cDNA was generated containing (using the cordon numbering of APP₆₉₅) the mutations K595N/M596L (the Swedish mutation) and V642F (the Indiana mutation). These and other APP mutations will generally be referred to herein, by the more common APP₇₇₀ codon numbering system i.e. for these two mutations, K670N/M671L (the Swedish mutations) and V717F (the Indiana mutation).

The double mutant APP₆₉₅ cDNA cassette was inserted into the cosmic expression vector, cosTet, which contains the Syrian hamster prion protein gene promoter. The vector was then microinjected into a mouse oocyte to create a transgenic line designated TgCRND8. These mice exhibit multiple diffuse amyloid deposits by three months of age, at which time deficits in spatial learning are apparent.

TgCRND8 mice have been crossed with various other transgenic mice bearing an AD-related mutation to product bi-transgenic mice, which show further, enhanced AD-related neuropathology.

### ADMINISTRATION AND MEDICAL USES

The statements below make reference to methods of treatment. It is to be understood that methods of treatment are not part of the invention, but the statements are retained as being relevant to the medical uses of the invention which are as defined in the appendant claims.

The present invention also includes methods of using Abeta₍₄₋₁₀₎ antigens to identify drugs that interfere with the binding of Abeta₄₂ to plaques. One such aspect includes drug-screening assays to identify drugs that mimic and/or complement the effect of Abeta₄₂. In one such embodiment, a drug library is screened by assaying the binding activity of a peptide comprising Abeta₍₄₋₁₀₎ to a specific small molecule. The effect of a prospective drug on the affinity of Abeta₄₂ to plaques is monitored. If the drug decreases the binding affinity of Abeta₄₂ to plaques, it becomes a candidate drug. Drugs can be screened for their ability to disrupt the plaque formation, hinder the fibrillogenesis process, or disaggregate preformed fibrils.

The antigens, antibodies or other compounds useful in the present invention can be incorporated as components of pharmaceutical compositions. The pharmaceutical compositions preferably contain a therapeutic or prophylactic amount of at least one of the antigens, antibodies or other compounds thereof with a pharmaceutically effective carrier.

In preparing the pharmaceutical compositions useful in the present methods, a pharmaceutical carrier should be employed which is any compatible, nontoxic substance suitable to deliver the, antigens, antibodies or binding fragments thereof or therapeutic compounds identified in accordance with the methods disclosed herein to the patient. Sterile water, alcohol, fats, waxes, inert solids and even liposomes may be used as the carrier. Pharmaceutically acceptable adjuvants (buffering agents, dispersing agents) may also be incorporated into the pharmaceutical composition. The antibodies and pharmaceutical compositions thereof are particularly useful for parenteral administration, i.e., intravenously, intraarterially, intramuscularly, or subcutaneously. However, intranasal or other aerosol formulations are also useful. The concentration of compound such as an antibody in a formulation for administration can vary widely, i.e., from less than about 0.5%, usually at least 1% to as much as 15 or 20% or more by weight, and will be selected primarily based on fluid volumes, viscosities, etc., preferred for the particular mode of administration selected. Actual methods for preparing administrable compositions will be known or apparent to those skilled in the art and are describe in more detail in, for example, Remington's Pharmaceutical Science, 18th Ed., Mack Publishing Co., Easton, Pa. (1990).

Immunogenic compositions of the present invention, or antibodies or antigen binding fragments of the present disclosure are administered at a therapeutically effective dosage sufficient to modulate amyloid deposition (or amyloid load) in a subject. A "therapeutically effective dosage" preferably modulates amyloid deposition by at least about 20%, more preferably by at least about 40%, even more preferably by at least about 60%, and still more preferably by at least about 80% relative to untreated subjects. The ability of a method to modulate amyloid deposition can be evaluated in model systems that may be predictive of efficacy in modulating amyloid deposition in human diseases, such as animal model systems known in the art (including, e.g., the method described in PCT Publication WO 96/28187) or by in vitro methods, .e.g., the method of Chakrabartty, described in PCT Publication WO 97/07402, or the TgCRND8 model system described herein. Furthermore, the amount or distribution of amyloid deposits in a subject can be non-invasively monitored in vivo, for example, by use of radiolabelled tracers which can associate with amyloid deposits, followed by scintigraphy to image the amyloid deposits (see, e.g., Aprile, C. et al., Eur. J. Nuc. Med. 22:1393 (1995); Hawkins, P. N., Baillieres Clin. Rheumatol. 8:635 (1994) and references cited therein). Thus, for example, the amyloid load of a subject can be evaluated after a period of treatment according to the methods disclosed and compared to the amyloid load of the subject prior to beginning therapy with a therapeutic compound of the invention, to determine the effect of the therapeutic compound on amyloid deposition in the subject.

It will be appreciated that the ability of a method of the disclosure to modulate amyloid deposition or amyloid load can, in certain embodiments, be evaluated by observing the symptoms or signs associated with amyloid deposition or amyloid load in vivo. Thus, for example, the ability of a method of the present disclosure to decrease amyloid deposition or amyloid load may be associated with an observable improvement in a clinical manifestation of the underlying amyloid-related disease state or condition, or a slowing or delay in progregsion of symptoms of the condition. Thus, monitoring of clinical manifestations of disease can be useful in evaluating the amyloid-modulating efficacy of a method of the disclosure.

The methods of the present disclosure may be useful for treating amyloidosis associated with other diseases in which amyloid deposition occurs. Clinically, amyloidosis can be primary, secondary familial or isolated. Amyloids have been categorized by the type of amyloidogenic protein contained within the amyloid. Nonlimiting examples of amyloids which can be modulated, as identified by their amyloidogenic protein, are as follows (with the associated disease in parentheses after the amyloidogenic protein): beta-amyloid (Alzheimer's disease, Down's syndrome, hereditary cerebral hemorrhage amyloidosis [Dutch], cerebral angiopathy) ; amyloid A (reactive [secondary] amyloidosis, familial Mediterranean Fever, familial amyloid nephropathy with urticaria and deafness [Muckle-Wells syndrome]); amyloid kappa L-chain or amyloid lambda L-chain (idiopathic [primary], myeloma or macroglobulanemia-associated); Abeta2M (chronic hæmodialysis); ATTR (familial amyloid polyneuropathy [Portuguese, Japanese, Swedish], familial amyloid cardiomyopathy [Danish], isolated cardiac amyloid, systemic senile amyloidosis); AIAPP or amylin (adult onset diabetes, insulinoma); atrial naturetic factor (isolated atrial amyloid); procalcitonin (medullary carcinoma of the thyroid); gelsolin (familial amyloidosis [Finnish]); cystatin C (hereditary cerebral hemorrhage with amyloidosis [Icelandic]); AApoA-I (familial amyloidotic polyneuropathy [Iowa]); AApoA-II (accelerated senescence in mice); fibrinogen-associated amyloid; lysozyme-associated amyloid; and AScr or PrP-27 (Scrapie, Creutzfeldt-Jacob disease, Gerstmann-Straussler-Scheinker syndrome, bovine spongiform encephalitis).

The following examples are offered by way of illustration, not by way of limitation.

### DETAILED DESCRIPTION OF THE PREFERRED EMBODIMENTS

### Example 1

### Antigen Synthesis and Structural Characterization

In this example, the inventors describe how to synthesize, purify and characterize synthetic Abeta peptide immunogens.

Syntheses of the following Abeta peptides: Abeta₄₂, Abeta₄₀, Abeta₃₀, and N-terminal epitope peptides were performed with an ABIMED EPS-221 semi-automated peptide synthesizer, using NovaSyn (Novabiochem)PEG graft polymer resin and Fmoc N-terminal protection methodology as described. See Mayer-Fligge et al., J. Pept. Sci. 4:355-363 (1998). Fmoc-deprotection steps and final deprotection cycles were monitored spectrophotometrically. The synthetic peptides were purified using a semi-preparative, reverse phase, C18 µbondapak, HPLC column.

The molecular weights of the purified synthetic peptides were then characterized by plasma desorbtion (MALDI) and electrospray (ESI) mass spectroscopy. Only peptide fractions having-molecular masses corresponding to the predicted masses were used for the subsequent immunizations.

The secondary structure of the peptides in solution was evaluated using circular dichroism (CD) . The CD spectra were recorded using a JASCO J-500 spectropolarimeter. See Mayer-Fligge et al., J. Pept. Sci. 4:355-363 (1998). In addition, NMR studies were performed using 2D-NMR-NOESY analysis with a Bruker-AMX-600 instrument as previously described. Michels et al., "Structure and Functional Characterization of the periplasmis N-terminal polypeptide domain of the sugar specific ion channel protein (scry-porin), "Protein Science (in Press 2002)

### Example 2

### Immunization of CRND8 Mice with Abeta₄₂

In this example, the inventors show that the Abeta₄₂ peptide is immunogenic in mice expressing the APP transgene and in non-transgenic mice.

### Mice

The TgCRND8 mice have been described elsewhere by Chishti et al, J. Biol. Chem. 276:21562-21570 (2001). . The mice were maintained in an outbred C3H/C57BL/6J background which overexpresses the Beta-APP_{Swedish} and Beta-APP_{V717F} mutations in cis on the beta-APP₆₉₅ transcript . The Beta-APP_{Swedish} and Beta-APP_{V717F} genes were under the control of the Syrian hamster prion gene promoter. TgCRND8 mice derived from crosses of C3H/C57BL6 (82%/ 18%) transgene-positive hemizygous mice and wt CS7BL/6J mice were weaned, genotyped for the presence of the beta-APP transgene and housed in same-sex groups of 2-4 mice in standard mouse cages. The mice were provided with food pellets, powdered food, and water ad lib. All mice were handled for one week before the first immunization, and their weights were recorded the day before and two days after every immunizations. All of the experimental groups were sex and weight matched.

### Immunization Protocol and Sera Isolation

The synthetic Abeta₄₂ and a control peptide consisting of residues 8-37 (ATQRLANELVHSSNNFGAIL-SSTNVGSNTY) (SEQ ID NO:52) of the islet amyloid polypeptide (IAPP) peptides were isolated by reverse phase HPLC one a C18 µbondapak column and purity of the peptides was determined by mass spectrometry and amino acid analyses.

The immunization protocol and schedule were as previously described in Schenk et al. Nature 400:173-177 (1999). Next, antibody titers were determined in serum samples (200µl of blood) collected via the hind leg vein puncture at age 13 weeks, and by cardiac puncture at the cessation of the procedure, art 25 weeks of age. Prior to use in these studies, complement was deactivated by incubation at 56°C for 30 minutes. Ig fractions were isolated over a 5-ml protein G column, Samples were loaded, washed with PBS, eluted with 0.1 M NaCitrate and buffered with 1 M Tris. All Ig fractions were filter sterilized before use

### Immunization Results

Sera were isolated from non-immunized mice (N=18), and from both TgCRND8 mice and their non-transgenic littermates that had been repeatedly immunized over a 5-month period with either Abeta₄₂ (n=34; 18 TgCRND8 and 16 non-Tg), or with a peripheral amyloid peptide (islet associated polypeptide (IAPP), where the number of mice immunized was 17, with 10 being TgCRND8 and 7 non-transgenic (non-tg). The mice developed significant titers against Abeta₄₂ (1:5000-1:50,000) and against IAPP (1-5000 to 1:30,000). Interestingly, no significant differences were detected in the anti-Abeta₄₂ titers of TgCRND8 transgenic mice and their non-transgenic littermates. Every sample of sera from Abeta₄₂-immunized mice could positively stain mature Abeta plaques in histological sections of brain from 20-week-old non-immunized TgCRND8 mice. In contrast, the sera from the control peptide IAPP-immunized and non-immunized mice could not stain mature Abeta plaques in histological sections of brain from 20-week-old non-immunized TgCRND8 mice. Therefore, the results show that antibody autoimmunity can be induced which can recognize and bind to neuropathological plaques containing Abeta.

### EXAMPLE 3

### Inhibition of Fibril Formation by Mouse Immune Serum

In this example, as shown in Table 2, the inventors show that sera from most Abeta₄₂ immunized mice inhibited fibril formation.

At low concentrations solutions of Abeta peptides will spontaneously assemble into fibrils over a 14-day incubation period. These fibrils have a characteristic 50-70 Å diameter that can be monitored by electron microscopy as described below.

### Electron Microscopy

Abeta₄₂ was used directly after solubilization in water at a stock concentration of 10 mg/ml or after assembly into mature amyloid fibrils. Abeta₄₂ was incubated in the presence and absence of sera at a final peptide concentration of 100 µg/ml. Serial dilutions of various sera were added to Abeta₄₂ and incubated at Room Temperature (RT) for up to 2 wk. For negative stain electron microscopy, carbon-coated pioloform grids were floated on aqueous solutions of peptides. After the grids were blotted and air-dried, the samples were stained with 1% (w/v) phosphotungstic acid. The peptide assemblies were observed in a Hitachi 7000 electron microscope that was operated at 75V at a Magnification 60,000 X.

### Electron Microscopy Results

To assess the effect of Abeta immunized mouse sera on the assembly of Abeta into fibrils, sera were incubated as described above in the presence or absence of Abeta₄₂ at 37°C for up to 14 days. Aliquots from each reaction mixture were examined at days 1, 3, 7, 10 and 14 for the presence of Abeta₄₂ fibrils by negative stain electron microscopy.

In the absence of sera, or in the presence of non-immunized sera, Abeta₄₂ formed long fibrils (∼7500Å) with a characteristic 50-70 Å diameter. The long fibrils thus indicated that normal serum components did not inhibit Abeta fibril formation under the present assay conditions. In the presence of sera from IAPP-immunized animals, fewer long Abeta₄₂ fibrils were produced, but the fibrils that did form had the characteristic 50-70 Å diameter. In contrast, as shown in Table 2, the majority of Abeta₄₂-immunized mouse sera (n=27/34) largely blocked fibril formation, although a few sera (n=7/34) had little or no effect. Furthermore, Abeta-immunized sera from TgCRND8 mice or from non-transgenic littermates inhibited Abeta-fibril formation equivalently, indicating that the antibody repertoire is dependent only on the immunogen and not the load of endogenous Abeta₄₂.

As summarized in Table 2, no difference in the structure of the fibrils was detectable when incubated in the presence of non-immunized mouse sera. Sera from mice immunized with IAPP decreased the extent of fibril formation but fibrils that did form were similar to fibrils formed by Abeta₄₂ alone. Finally, sera from mice immunized with Abeta₄₂ inhibited fibrillogenesis to varying extents from complete inhibition to only slight decreases in fibril density. See Table 2.

**Table 2 Summary of Effects of Non-Immune, Abeta 42-immunized and IAPP immunized Sera on Fibril Formation, Fibril Disassembly and Cytotoxicity**

| **INHIBITION STUDIES** | | | | |
|---|---|---|---|---|
| Immunogen | Total Samples | Aggregation | Disaggregation | Toxicity |
| NonImmune | 18 | 0/18 | 0/18 | 0/18 |
| Abeta42 | 34 | 27/34 | 26/34 | 22/30 |
| IAPP | 17 | 4/17 | 1/.17 | 2/11 |

### Example 4

### Disruption of Existing Fibrils by Immune Serum

In this example, the inventors show that sera from Abeta₄₂-immunized mice disaggregated preformed Abeta₄₂ fibrils, but that preformed Abeta₄₂ fibrils are not affected by incubation with unimmunized control mouse sera or by sera from IAPP immunized mice.

In order to determine whether sera from Abeta₄₂ immunized mice can disrupt preformed Abeta fibrils, sera from Abeta₄₂ immunized mice were incubated with preformed Abeta₄₂ fibrils for up to 30 days. Abeta₄₂ fibrils, with evidence of aggregation, were generated by incubating Abeta aliquots at high concentrations with constant agitation. Incubation of preformed fibrils with no serum (Abeta alone), with IAPP-immunized sera, or with non-immunized sera (data not shown) had no effect, even after 30 days of incubation. In contrast, sera from Abeta₄₂-immunized mice (n=26/34) disaggregated Abeta₄₂ fibrils either to small short fibrils of 30Å diameter with an average length of 100Å, or to amorphous aggregates. This disaggregation was evident after only three days of incubation and was complete by 14 days. In addition, disaggregation was concentration-dependent, with increasing concentrations of antibody decreasing the time required for fibril disaggregation. Finally, because a 1:1 ratio of antibody to Abeta₄₂ was not necessary for disaggregation, it is likely that the anti-Abeta antibodies were binding only to a subset of Abeta species such as protofibillar oligomers or other precursors. The results were determined using electron microscopy as described in Example 4, at a magnification of 60,000X.

### EXAMPLE 5

### Mass Spectrometric Determination of the Immune Target Epitope of Abeta₄₂ Recognized by Mouse Antisera

In this example, the inventors show how to precisely identify an epitope having critical biological significance for use in therapy of amyloid deposit diseases.

### General Scheme

To elucidate the epitope recognized by the anti-Abeta₄₂-sera, high resolution Fourier-transform ion cyclotron resonance mass spectrometry (FT-ICR-MS; Marshall et al., Mass Spectrom. Rev. 17:1-35 (1998)) using both nano-electrospray (nESI) and MALDI-ionization was applied in combination with epitope excision and epitope extraction procedures discussed below. See Macht et al., Biochemistry 35: 15,633-15,639 (1996); Suckau et al., Proc. Natl Acad. Sci. USA 87:9848-9852 (1990); Przybylski et al., "Approaches to the characterization of tertiary and supramolecular protein structures by combination of protein chemistry and mass spectrometry." In New Methods for the study of Biomolecular Complexes, Kluwer Acad. Publ., Amsterdam, pp. 17-43 (1998).

In one procedure, known as epitope excision, we combined selective proteolytic cleavage of the intact, immobilized immune complex with mass spectrometric peptide mapping on the bound peptide after it was released. Specifically, antisera from Abeta₄₂-immunized TgCRND8 mice, control antisera from IAPP-immunized mice, mouse (monoclonal) and rabbit (polyclonal) Abeta₄₂-antibodies were immobilized in sepharose-microcapillaries. Next, the immobilized antibodies were exposed to Abeta₄₂ aggregates and allowed to bind the Abeta₄₂ epitope. The Epitope excision procedure of the immune complex was performed using a variety of proteases and exopeptidases, or with combinations of enzymes. See Table 2)

Alternatively, the epitope extraction procedure was used. For epitope extraction, Abeta₄₂ was predigested with the various proteases and, subsequently, the corresponding mixture of protease processed Abeta₄₂ peptides was applied to the antibody columns and the antibody was allowed to bind the epitope. The epitope was identified using mass spectroscopy upon elution of the bound peptide. This procedure was known as epitope extraction.

The individual procedures are described in detail below:

### Antibody Immobilization

A solution of 100 µg of coupling buffer (0.2 M NaHCO₃, 0.5 M NaCl, pH 8.3) was added to dry NHS-activated 6-aminohexanoic acid-coupled sepharose (Sigma), and the coupling reaction was performed for 60 min at 20 C. The sepharose material was then transferred onto a 100 µm microcapillary column that permits extensive washing without loss of material. See Macht et al., Biochemistry 35: 15,633-15,639 (1996). The column was washed alternatively using blocking buffer (ethanolamine/NaCl) and washing buffer (NaAc/NaCl) as described, and the column finally stored in PBS at pH 7.5, 4°C. See Macht et al., Biochemistry 35: 15,633-15,639 (1996).

### Epitope Excision

Epitope excision procedures were performed by first applying of 2-5 µg Abeta₄₂ or other Abeta-antigens to the antibody microcolumn and incubating for 60 min at 20°C with gentle shaking. After successive washes with 5 x 4 ml PBS, protease digestion was performed on the column for 2 h at 37°C by incubating 0.2 µg of protease in 200 µl PBS. The proteases included trypsin; Lys-C protease; Asp-N-protease; α-chymotrypsin; and Glu-C protease. The unbound and digested peptides or supernatant were removed by washing with 5 × 4 ml PBS. Next, the antibody bound epitope peptide was disassociated and eluted by the addition of 500 µl 0.1% (v/v) TFA (epitope elution) . After incubation for 15 min at 20°C the epitope elution fraction was lyophilized and reconstituted in 10 µl 0.1% TFA for mass spectrometric analysis. Procedures with additional exopeptidase digestion were performed by incubation with 0.1 µg aminopeptidase M or carboxypeptidase Y for 30 min, followed by washing with 5 × 4 ml PBS.

### Epitope Extraction

The epitope extraction procedure was performed in the same manner as epitope elution, except that the proteolytic digest mixture was applied to the antibody column and incubated for 60 min at 20°C. Subsequently, the unbound peptides (supernatant) were removed by washing with 5 x 4 ml PBS. Next, the antibody bound epitope was disassociated and eluted by the addition of 500 µl 0.1% (v/v) TFA (epitope elution). After incubation for 15 min at 20°C the epitope elution fraction was lyophilized and reconstituted in 0 µl 0.1% TFA for mass spectrometric analysis.

### Proteolytic Digestion

Proteolytic digestions of free antigens were carried out with 5-50 µg peptide dissolved in 50 mM NH₄HCO₃ for 2 h at 37°C at a substrate-to-protease ratio of 50:1. The reaction mixtures were lyophilized for mass spectrometric analysis or prepared for epitope extraction. The proteases used were trypsin (Promega, Madison); Lys-C, Asp-N, Glu-C (Roche-Boehringer Mannheim); α-chymotrypsin, aminopeptidase M, carboxypeptidase Y (Sigma).

### Mass Spectrometry

FTICR-MS was performed with a Bruker (Bruker Daltonik, Bremen, FRG) Apex II FTICR spectrometer equipped with a 7T superconducting magnet and ICR analyzer cell. See Bauer et al., Anal. Biochem. 298:25-31 (2001). The MALDI-FTICR source with pulsed collision Apollo-nano-ESI-source, and instrumental conditions and mass calibration were described previously. See Fligge et al., Biochemistry 39: 8491-8496 (2000). Mass determination accuracies were ∼1 ppm (MALDI) and typically, 0.5-1 ppm (ESI) at a mass resolution of ∼200,000. 2,5-Di-hydroxybenzoic acid (DHB) was used as matrix for MALDI-MS sample preparation. See Bauer et al., Anal. Biochem. 298:25-31 (2001). ESI-MS was generally performed with aqueous 0.01% TFA solutions. See Fligge et al., Biochemistry 39: 8491-8496 (2000).

### Mass Spectroscopy Results

Epitope excision and extraction with the antibody immobilized to a sepharose-conditioned microcapillary was used, with analyses by ESI- and MALDI-FTICR-mass spectrometry. See Macht et al. Biochemistry 35:15633-39 (1996); Fligge et al., Biochemistry 39: 8491-8496 (2000); See Bauer et al., Anal. Biochem. 298:25-31 (2001); Przybylski et al., "Approaches to the characterization of tertiary and supramolecular protein structures by combination of protein chemistry and mass spectrometry." In New Methods for the study of Biomolecular Complexes, Kluwer Acad. Publ., Amsterdam, pp.17-43 (1998). First, MALDI-MS of tryptic peptide mixture of free Abeta₄₂ antigen shows all of the expected Abeta proteolytic peptides including the following:

### Peptide Mass (Da)

1. Abeta(₁₋₁₆) 1954.8892
2. Abeta₍₆₋₁₆₎ 1336.6030
3. Abeta₍₁₇₋₂₈₎ 1325.6735
3. Abeta₍₂₉₋₄₂₎ 1268.7804
5. Abeta₍₁₇₋₄₂₎ 2575.4164

Epitope excision, using Lys-C and trypsin digestions, eluted a single peptide fragment which produced a single ion species Abeta₍₁₋₁₆₎ 1954.8806 using MALDI-FTICR detection. In this case, the R5 residue of Abeta was being shielded from digestion by Lys-C and trypsin.

The peptide fragment Abeta₍₁₋₁₁), 1324 . 5395 Da eluted upon epitope excision with *S. Aureus* Glu-C protease.

ESI- and MALDI spectra of the eluate from epitope extraction after α-chymotrypsin and aminopeptidase M cleavage produced fragments Abeta_{(1 10)} 1195.4968 Da and Abeta₍₄₋₁₀₎ 880.3827 Da.

The core epitope was determined by using aminopeptidase M-digestion of the antibody bound chymotryptic fragment and Abeta₍₁₁₀₎ immune complex. This double digestion identified Abeta₍₄₋₁₀), FRHDSGY as the minimal epitope with comparable affinity to that of Abeta₄₂. The C-terminal amino acid is Y10 because further C-terminal digestion from Y10 using carboxypeptidase A yielded peptides having drastically diminished affinity as compared to Abeta₄₂.

Table 3 shows the peptide fragments that were obtained by mass spectroscopy of the epitope excision and extraction procedures using the anti-Abeta antibodies and Abeta peptides. When the Abeta₄₂ peptide (Table 3, row 1) was predigested with trypsin, the peptide obtained from the antibody binding site corresponded to the sequence shown in Table 3, row 1. The combination of trypsin and Lys-C proteases identified the same 16 residue peptide (Table 3, row 2). When the protease was S. Aureus Glu-C protease and it was used in epitope excision, an 11 residue peptide was eluted from the antibody binding site, as shown in row 3. A ten residue peptide was observed with α-chymotrypsin alone digestion (Table 3, row 4). As shown in Table 3, row 5, the seven amino acid core epitope was observed when the protease digestions were performed with the two enzymes α-chymotrypsin and aminopeptidase M.

**Table 3 Peptides Identified by Mass Spectroscopy**

| Row No. | Number of Residues | | | | Proteases Used |
|---|---|---|---|---|---|
| | 1 | 5 | 10 | 15 | |
| 1 | D A E F R H D S G Y E V H H Q K SEQ ID NO: 22 | | | | trypsin |
| 2 | D A E F R H D S G Y E V H H Q K SEQ ID NO: 22 | | | | trypsin and lys-C-protease |
| 3 | D A E F R H D S G Y E S. SEQ ID NO: 23 | | | | Aureus Glu-C protease |
| 4 | D A E F R H D S G Y SEQ ID NO: 24 | | | | α-chymotrypsin |
| 5 | F R H D S G Y SEQ ID NO : 1 | | | | α-chymotrypsin and aminopeptidase M |

### Summary

MALDI- and ESI-MS analysis identified a linear epitope comprising the N-terminal sequence, Abeta₍₁₋₁₀) as the only, specific product upon epitope excision. See Tables 3 and 4. Mass spectroscopy of a typsin digestion of the free Abeta₄₂ antigen yielded all expected peptides, (1-16), (6-16), (17-28), 29-42). See Tables 3 and 4. Epitope excision with trypsin and Lys-C-protease provided a single peptide (1-16). Glu-C-protease and α-chymotrypsin generated only the fragments (1-11) and (1-₁₀₎, respectively. See Tables 3 and 4. In contrast, residues R5, E3, F4 were shielded from digestion with these proteases, respectively. Further digestion of antibody-bound endoprotease fragments were performed with exopeptidases to define the core epitope. Aminopeptidase M-digestion of the chymotryptic fragment identified Abeta₍₄₋₁₀₎; FRHDSGY as the minimal epitope with comparable affinity to that of Abeta₄₂, while further C-terminal digestion from Y10 (carboxypeptidase A) yielded drastically diminished affinity. Affinity differences obtained in the mass spectrometric epitope excision experiments were entirely consistent with affinities determined by ELISA of the synthetic epitope peptides biotinylated at the N-terminus via an alkylamido-spacer group. See Gitlin et al., Biochem. J. 242:923-926 (1987); Craig et al., Anal. Chem. 68:697-701 (1996). The epitope was identified unequivocally by the high mass determination accuracy (0.5 - 2 ppm) of the monoisotopic molecular ions. In addition, these results were confirmed by sequence-specific fragmentation of selected molecular ions in FTICR-spectra by IR-multiphoton laser dissociation, and by control experiments with sequence mutants and homologous Abeta₄₂ peptides (data not shown). See Fligge et al., Biochemistry 39: 8491-8496 (2000). Thus, rat Abeta₄₂, which contains an R5G and Y10F double mutation yielded no elution product upon epitope excision. In contrast, human Abeta₍₁₋₄₀₎ and Abeta₍₁₋₃₀₎ provided the same epitope (4-10) as Abeta₄₂. The control antibody from IAPP-immunized mice yielded no detectable epitope peptide. See Tables 3 and 4.

**Table 4 Summary Of Mass Spectrometric Epitope Excision/ Extraction Data For Aβ42-Immunised Sera And IAPP- Immunised Sera.**

| Epitope experiment^{a} | Protease | Peptides identified^{c} | | | |
|---|---|---|---|---|---|
| | | A 2-antisera | | L4,PP-antisera^{c} | |
| | | Supernatant | Elution | Supernatant | Elution |
| | | fraction | | fraction | |
| excision | Lys-C | 17-28 29-42 | 1-16 | 1-16 17-28 29-42 | -^{d} |
| | Trypsin | 17-28 29-42 | 1-16 | 1-5 6-16 17-28 29-42 | - |
| | Glu-C | 12-22 23-42 | 1-11 | 4-11 12-22 23-42 | - |
| | Asp-N | 23-42 | 2-22 | 2-22 23-42 | - |
| extraction | Trypsin | 1-5 6-16 17-28 29-42 | 1-16 | 1-5 6-16 17-28 29-42 | - |
| | a-chymotrypsin | 5-10 11-20 21-42 | 1-10 | 1-4 5-10 11-20 21-42 | - |
| | a-chymotrypsin/Apase-M | 1-4 5-10 11-20^{e} | 4-10 | nd | - |
| | Trypsin/Apase-M | 6-16 7-16^{e} | 4-16 | nd | - |

| | | | | | |
|---|---|---|---|---|---|
| ^{a} Epitope- excision and -extraction (s. *Methods* and text) ^{b} Concentration of proteases as given in *Methods;* Apase-M, microsomal aminopeptidase. ^{c} Sequences of major peptides identified in supernatant and epitope fractions upon elution with TFA ^{d} No detectable biding of Aß- sequences. ^{e} Only N- terminal peptides are given. | | | | | |

### Example 6

### Structural Characterization of Abeta Peptides

In this example, the inventors compared the affinity of the identified synthetic epitope peptides with that of Abeta₄₂ for the immobilized antibodies and characterized the secondary structure of the synthetic epitope peptides in solution.

The epitope identified by mass spectrometry was further characterized using synthetic peptides, secondary structural analysis and immuno-analytical characterization of the corresponding authentic peptides, biotin-Gly-Gly-Abeta₍₁₋₁₀₎ and biotin-Abeta₍₄₋₁₀₎. First, the affinity of the various peptides for anti-Abeta antibody was estimated using ELISA and dot-blot analysis of the epitope peptides (data not shown)). The results showed that all of the peptides shown in Table 3 displayed comparable affinity to Abeta₄₂.

To evaluate a possible conformational effect of the active epitope, a secondary structural comparison of the N-terminal peptides with the previously reported structures of Abeta₄₀ and Abeta₄₂ was performed. The CD spectra and 2D NMR-NOESY spectra (data not shown) of the N-terminal, polar peptides Abeta₍₁₋₁₀₎ and Abeta₍₁₋₁₆₎ do not show any evidence of a definite solution structure for the Abeta fragments. Such data suggests, however, a certain flexibility of the epitope for antibody recognition. This is consistent with the secondary structure prediction for the Abeta₄₂ sequence showing a break in the propensity for α-helix formation around the Abeta(₄₋₁₀) epitope region. In contrast, α-helix propensity and helix-coil/β-sheet conformational transition were observed for sequences comprising the transmembrane region (Abeta₍₁₈₋₄₂₎). See Coles et al . , Biochemistry 37: 11064-11077 (1998); Kohno et al., Biochemistry 35: 16094-16104 (1996).

### Example 7

### Effect of Sera on Abeta-induced Toxicity

In this example, the inventors evaluated the ability of Abeta-immunized sera to inhibit Abeta 42-induced cytotoxicity.

### General Scheme

To explore whether the prevention of memory deficits in TgCRND8 mice after Abeta-immunization might reflect a similar effect on the cytoxicity of Abeta, we performed standard Abeta₄₂ toxicity assays using PC-12 cells. See McLaurin et al., J. Biol. Chem. 275:18495-502 (2000); Pallitto et al., Biochemistry 38:3570-78 (1999). First, PC12 cells were incubated with Abeta₄₂, in the presence or absence of sera for 24 hours. Next, cellular toxicity was measured using both the Alamar blue assay (Ahmed et al., J. Immunol. Methods 170:211-24 (1994)), which is indicative of metabolic activity, and the Live/Dead assay (Pike et al., J: Biol. Chem. 270:23895-98 (1995)), which indicates both intracellular esterase activity and plasma membrane integrity.

### Abeta Toxicity Assay

PC-12 cells were plated at 500 cells per well in a 96 well plate and suspended in 30 ng/ml NGF (Alamone Labs, Israel) diluted in N2/DMEM (Gibco/BRL, Rockville, MD). Cells were allowed to differentiate for 5-7 days to a final cell number of 10,000-15,000 per well. Abeta was maintained in solution (25 micromolar) for 3 days at RT to induce fibrillogenesis before addition to cultures. This Abeta preparation contains a multitude of assembly oligomers including, ADDLs and protofibrils (Abeta-species so far identified as neurotoxic) as determined by electron microscopy (data not shown). See Lambert et al., J. Neurochem. 79:595-605 (2001); Walsh et al., J. Biol. Chem., 274:25945-52 (1999); Hartley et al., J. Neuroscience 19:8876-8884 (1999). In addition, western blot analyses demonstrated that Abeta₄₂-immunised sera recognizes Abeta₄₂ monomers, tetramers, hexamers and large oligomers of greater than 98 kDa (data not shown). After the 3 day pre-incubation, Abeta was added to cell cultures at a final concentration of 0.1 µg/µl and incubated for 24 hrs at 37°C. Next, toxicity was assayed using the Live/Dead fluorescent assay (Molecular Probes, Eugene, OR) and Alamar Blue Assay (Biosource Inc, Camarillo, CA).

### Results

The Sera from non-immunized or IAPP-immunized mice had no effect on Abeta-toxicity. In contrast, sera isolated from Abeta₄₂-immunized mice prevented Abeta₄₂-cytotoxicity in a concentration-dependent manner, but displayed a marked variability in the extent of this effect. In this assay n=18/22, p<0.01 and n=4/22, p<0.001 in comparison to Abeta42-induced toxicity. The correlation between cell survival and the extent of fibril disaggregation was plotted for individual sera and revealed a direct correlation between the effectiveness of sera to inhibit toxicity and disaggregate fibrils. Moreover, antibodies that were the most effective at inhibiting fibril formation/disaggregation were also the most effective in reducing toxicity (Day 3 p<0.001 and Day 7 p<0.0001 in comparison to inactive sera).

The stoichiometry of antibody to Abeta necessary to prevent cytotoxicity could provide insight into the mechanism of action. In order to determine the stoichiometry of antibody to Abeta necessary to elicit the inhibition of cytotoxicity, we determined the EC₅₀ for 10 reactive sera. The EC₅₀ values ranged from 1:100-1:300 with a mean ± SD of 234±39, when the EC₅₀ is defined as the amount of sera that rescued 50% of the Abeta-induced cytotoxicity. As a result, we found that the protective effect was detected at low antibody to Abeta ratios, 50:1, suggesting that the antibodies were binding to a low abundance species of Abeta such as Abeta-oligomers, protofibrils, or precursor protein fragments, rather than monomeric Abeta or Abeta aggregates. Furthermore, active sera caused a significant decrease in Abeta-cytotoxicity at all doses tested; suggesting that cell death was induced by the processes specifically blocked by the sera. Statistical analyses was accomplished using one way ANOVA with Fischer's PLSD * p<0.01 and † p<0.001.

### Example 8

### Serum Components Mediating Protective Effect

In this example the inventors show how to determine which serum components were responsible for the reduced cytotoxicity of Abeta. The inventors found that the active component was in the purified IgG fraction from sera and no other serum component could inhibit of Abeta mediated cell death.

In order to verify that the effects of Abeta-immunization were due to Abeta-induced antibodies, rather than due to some other effect, e.g. secondary changes in the expression of other serum proteins. Therefore, to confirm that only antibodies selectively targeting the Abet(4-10) epitope were effective, we performed cytotoxicity experiments using purified IgG fractions from Abeta₄₂-immunized sera. In addition, we included the commerically available monoclonal antibodies, 4G8, 6E10 and Bam10 having specificity for particular epitopes of Abeta.

The results were conclusive. The immunoglobulin G purified from Abeta₄₂-immunized sera demonstrated the same inhibition of toxicity as crude sera, suggesting that other serum components did not contribute to the protective response. Furthermore, these IgG fractions inhibited Abeta-fibrillogenesis and induced Abeta fibril disaggregation to the same extent as whole sera. The antibodies 4G8 and 6E10, which recognize Abeta sequences 17-24 and 11-17 respectively, do not inhibit fibrillogenesis but do decrease the amount of total fibril. The latter effect may arise because these antibodies will bind to a small portion of the free Abeta peptide in solution, thereby sequestering it from fibril formation. In contrast, Bam10, which recognizes a sequence within Abeta₁₋₁₀, inhibits fibril formation similar to that shown with the Abeta₄₂-immunized sera. These results further demonstrate both that only antibodies that recognize the N-terminal of Abeta sequence are effective inhibitors of fibrillogenesis, and that the active component within the Abeta₄₂-immunized sera is a specific IgG.

### Examples 9 - 27

### ANTIGEN DESIGN

The peptides shown in Table 5 and Examples 9-27 are designed according to the formula shown below:

I. (A) ₙ -- (Th)ₘ -- (B)ₒ -- Abeta(₄₋₁₀) -- (C)ₚ

Where a single copy of Abeta₍₄₋₁₀₎ is present and n is 0, m is 1, o is 2, B is glycine, C is glycine, p is 1, and the T-cell helper eptitope is any of SEQ ID NO: 3, 4, 5, 6, 7, 8, 9, 10, 11, 12, 13, 14, 15, 16, 17, 18, 19, 20, or 21. These combined B and T cell epitope containing antigens correspond to SEQ ID NO: 25, 26, 27, 28, 29, 30, 31, 32, 33, 34, 35, 36, 37, 38, 39, 40, 41, 42 and 43.

**TABLE 5 Abeta Peptide Antigens**

| **Example** | **SEQ ID NO:** | **ANTIGEN PEPTIDE SEQUENCE** |
|---|---|---|
| 9 | 25 | FFLLTRILTIPQSLD-GG**FRHDSGYG** |
| 10 | 26 | KKLRRLLYMIYMSGLAVRVHVSKEEQYYDY-GG**FRFHDSGYG** |
| 11 | 27 | KKQYIKANSKFIGITE-GG**FRHDSGY**G |
| 12 | 28 | KKFNNFTVSFWLRVPKVSASHL-GG**FRHDSGY**G |
| 13 | 29 | YMSGLAVRVHVSKEE-GG**FRHDSGY**G |
| 14 | 30 | YDPNYLRTDSDKDRFLQTMVKLFNRIK-GG**FRHDSGY**G |
| 15 | 31 | GAYARCPNGTRALTVAELRGNAEL-GG**FRHDSGY**G |
| 16 | 32 | LSEIKGVIVHRLEGV-GG**FRHDSGY**G |
| 1 7 | 33 | GILESRGIKARITHVDTESY-GG**FRHDSGY**G |
| 18 | 34 | WVRDIIDDFTNESSQKT-GG**FRHDSGY**G |
| 19 | 35 | DVSTIVPYIGPALNHV-GG**FRHDSGY**G |
| 20 | 36 | ALNIWDRFDVFCTLGATTGGYLKGNS-GG**FRHDSGY**G |
| 21 | 37 | DSETADNLEKTVAALSILPGHGC-GG**FRHDSGY**G |
| 22 | 38 | EEIVAQSIALSSLMVAQAIPLVGELVDIGFAATNFVESCGG**FRHDSGY**G |
| 23 | 39 | DHEKKHAKMEKASSVFNVVNS-GG**FRHDSGY**G |
| 24 | 40 | KWFKTNAPNGVDEKHRH-GG**FRHDSGY**G |
| 25 | 41 | GLQGKHADAVKAKG-GG**FRHDSGY**G |
| 26 | 42 | GLAAGLVGMAADAMVEDVN-GG**FRHDSGY**G |
| 27 | 43 | STETGNQHHYQTRWSNANK-GG**FRHDSGY**G |
| 28 | 44 | STETGNQHHYQTRWSNANK-G**FRHDSGY**G |
| 29 | 45 | STETGNQHHYQTRWSNANK-**FRHDSGY**G |
| 3 0 | 46 | STETGNQHHYQTRVVSNANK- **FRHDSGY** |
| 31 | 47 | GG**FRHDSGY**GG-STETGNQHHYQTRWSNANK |
| 32 | 48 | GG**FRHDSGY**G-STETGNQHHYQTRWSNANK |
| 33 | 49 | GG**FRHDSGY**-STETGNQHHYQTRWSNANK |
| 34 | 50 | **FRHDSGY**GG-STETGNQHHYQTRWSNANK |
| 35 | 51 | **FRHDSGY**G-STETGNQHHYQTRWSNANK |

### Example 28

### ANTIGEN DESIGN

The peptide shown in Example 28 (Table 5), corresponding to SEQ ID NO: 44 is an example where n is 0, m is 1, o is 1, B is glycine, C is glycine, p is 1, and the T-cell helper eptitope is SEQ ID NO: 21. The combined B and T cell epitope containing antigen corresponds to the peptide shown in SEQ ID NO: 44.

### Example 29

### ANTIGEN DESIGN

The peptide shown in Example 29, (Table 5), corresponding to SEQ ID NO: 45 is an example where n is 0, m is 1, o is 0 and the T cell epitope is connected to the B cell epitope directly through a peptide bond, C is glycine, p is 1, and the T-cell helper eptitope is SEQ ID NO: 21. The combined B and T cell epitope containing antigen corresponds to the peptide shown in SEQ ID NO: 45.

### Example 30

### ANTIGEN DESIGN

The peptide shown in Example 30, (Table 5), corresponding to SEQ ID NO: 46 is an example where n is 0, m is 1, o is 0 and the T cell epitope is connected to the B cell epitope directly through a peptide bond, C is glycine, p is 1, and the T-cell helper eptitope is SEQ ID NO: 21. The combined B and T cell epitope containing antigen corresponds to the peptide shown in SEQ ID NO: 46.

### Examples 31-33

### ANTIGEN DESIGN

The peptides shown in Examples 31-33 (Table 5), are designed according to formula II shown below:

II. (A)ₙ -- Abeta₍₄₋₁₀₎ -- (B)ₒ -- (Th)ₘ -- (C)ₚ

Where a single copy of Abeta₍₄₋₁₀₎ is present and n is 2, m is 1, o is 2, A and B are glycine, and p is 0, and the T-cell helper eptitope is SEQ ID NO: 21. These combined B and T cell epitope containing antigens correspond to SEQ ID NO: 47, 48 and 49.

### Example 34 & 35

### ANTIGEN DESIGN

The peptide shown in Examples 34 (Table 5), are designed according to formula II shown below:

II. (A) ₙ -- Abeta₍₄₋₁₀₎ -- (B)ₒ -- (Th)ₘ -- (C)ₚ

Where a single copy of Abeta₍₄₋₁₀₎ is present and n is 0, m is 1, o is 2 in Example 34 and o is 1 in Example 35, B is glycine, and p is 0, and the T-cell helper eptitope is SEQ ID NO: 21. These combined B and T cell epitope containing antigens correspond to SEQ ID NO: 50 and 51.

### Example 36

### Synthesis of Designed Peptides

Solid phase peptide syntheses of the designed peptides corresponding to SEQ ID NO: 25, 26, 27, 28, 29, 30, 31, 32, 33, 34, 35, 36, 37, 38, 39, 40, 41, 42, 43, 45, 46, 47, 48, 49, 50, 51 and a control peptide, islet amyloid polypeptide (IAPP) (SEQ ID NO: 52) are performed on a 100 µmole scale using manual solid-phase synthesis and a Symphony Peptide Synthesizer using *Fmoc* protected Rink Amide MBHA resin, *Fmoc* protected amino acids, O-benzotriazol-1-yl-N,N,N', N-tetramethyl-uronium hexafluorophosphate (HBTU) in N,N-dimethylformamide (DMF) solution and activation with N-methyl morpholine (NMM), and piperidine deprotection of *Fmoc* groups (Step 1). When required, the selective deprotection of the Lys(Aloc) group is performed manually and accomplished by treating the resin with a solution of 3 eq of Pd(PPh₃)₄ dissolved in 5 mL of CHCl₃:NMM:HOAc (18:1:0.5) for 2 h (Step 2). The resin is then washed with CHC₃ (6 X 5 mL), 20% HOAc in Dichloromethane (DCM) (6 X 5 mL), DCM (6 X 5 mL), and DMF (6 X 5 mL). In some instances, the synthesis is then re-automated for the addition of one AEEA (aminoethoxyethoxyacetic acid) group, the addition of acetic acid or the addition of a 3-maleimidopropionic acid (MPA) (Step 3). Resin cleavage and product isolation is performed using 85% TFA/5% TIS/5% thioanisole and 5% phenol, followed by precipitation by dry-ice cold Et₂O (Step 4). The products are purified by preparative reversed phased HPLC using a Varian (Rainin) preparative binary HPLC system: gradient elution of 30-55% B (0.045% TFA in H.sub.2.O (A) and 0.045% TFA in CH₃ CN (B) ) over 180 min at 9.5 mL/min using a Phenomenex Luna 10 µ phenyl-hexyl, 21 mm X 25 cm column and UV detector "(Varian Dynamax UVD II) at 214 and 254 nm. Purity and mass verification is determined 95% by RP-HPLC mass spectrometry using a Hewlett Packard LCMS-1100 series spectrometer equipped with a diode array detector and using electro-spray ionization.

### Example 37

### Immunization of CRND8 Mice with Peptide Antigens Designed According to Formulas I and II

TgCRND8, mice as described in Example 2 are waned and genotyped for the presence of the beta-APP transgene and housed in same-sex groups of 2-4 mice in standard mouse cages. The mice are provided with food pellets, powdered food, and water *ad lib*. All mice are handled for one week before the first immunization, and their weights are recorded the day before and two days after every immunization. All of the experimental groups are sex and weight matched.

### Immunization Protocol and Sera Isolation

Synthetic peptides corresponding to SEQ ID NO:25, 26, 27, 28, 29, 30, 31, 32, 33, 34, 35, 36, 37, 38, 39, 40, 41, 42, 43, 45, 46, 47, 48, 49, 50, 51 and a control peptide, islet amyloid polypeptide (IAPP) peptide (SEQ ID NO: 52) are used to immunize transgenic CRND8 mice. The immunization protocol and schedule are as previously described in Schenk, et al. Nature 400:173-177 (1999). Each Peptide is freshly prepared f rom lyophilized powder for each set of injections. For immunizations, 2 mg of each peptide is added to a separate container of 0.9 ml deionized water and the mixtures are vortexed to mix the solutions. Next, 100 µl of 10X phosphate buffered saline (PBS) (where 1X PBS is 0.15 M NaCl, 0.01 sodium phosphate at pH7.5) is added to each peptide solution. Each solution is again vortexed and allowed to sit overnight at 37°C. The peptides are emulsified in a 1:1 (v/v) ratio with Complete Fruend's adjuvant for the first immunization and Freund's incomplete adjuvant for subsequent boosts. The first boost is two weeks after the initial immunization and monthly thereafter. Each animal is immunized with about 100 µg of antigen per injection. Each immunization group contains from 6 to 10 mice. Next, antibody titers are determined in serum samples (200µl of blood) collected via the hind leg vein puncture at age 13 weeks, and by cardiac puncture at the cessation of the procedure, at 25 weeks of age. Prior to use in these studies, complement is deactivated by incubation at 56°C for 30 minutes. Ig fractions are isolated over a 5-ml protein G column. Samples are loaded, washed with PBS, eluted with 0.1 M NaCitrate and buffered with 1 M Tris. All Ig fractions are filter sterilized before use.

### Immunization Results

Sera are isolated from mice immunized with synthetic peptides corresponding to SEQ ID NO: 25, 26, 27, 28, 29, 30, 31, 32, 33, 34, 35, 36, 37, 38, 39, 40, 41, 42, 43, 45, 46, 47, 48, 49, 50, 51 and a control peptide, islet amyloid polypeptide (IAPP) peptide (SEQ ID NO:52) and from non-immunized TgCRND8 mice and their non-transgenic littermates.

Most mice develop significant titers against Abeta₄₂, the immunogen or against IAPP. Interestingly, no significant differences are detected in the anti-Abeta₄₂ titers of TgCRND8 transgenic mice and their non-transgenic littermates. The sera from immunized mice are used to positively stain mature Abeta plaques in histological sections of brain from 20-week-old non-immunized TgCRND8 mice. In contrast, the sera from the control peptide IAPP-immunized and non-immunized mice are not able to stain mature Abeta plaques in histological sections of brain from 20-week-old non-immunized TgCRND8 mice. Therefore, the results show that antibody autoimmunity can be induced which can recognize and bind to neuropathological plaques containing Abeta.

### EXAMPLE 38

### Inhibition of Fibril Formation by Mouse Immune Serum

As discussed in Example 3, Abeta peptides will spontaneously assemble into fibrils over a 14-day incubation period and the fibrils have a characteristic 50-70 Å diameter that can be monitored by electron microscopy as described below.

### Electron Microscopy

Abeta₄₂ is used directly after solubilization in water at a stock concentration of 10 mg/ml or after assembly into mature amyloid fibrils. Abeta₄₂ is incubated in the presence and absence of sera from mice immunized with peptide antigens corresponding to SEQ ID NO: 25, 26, 27, 28, 29, 30, 31, 32, 33, 34, 35, 36, 37, 38, 39, 40, 41, 42, 43, 45, 46, 47, 48, 49, 50, 51 and a control peptide, islet amyloid polypeptide (IAPP) peptides at a final peptide concentration of 100 µg/ml. Serial dilutions of the sera are added to Abeta₄₂ and incubated at Room Temperature (RT) for up to 2 wk. For negative stain electron microscopy, carbon-coated pioloform grids are floated on aqueous solutions of peptides. After the grids are blotted and air-dried, the samples are stained with 1% (w/v) phosphotungstic acid. The peptide assemblies are observed in a Hitachi 7000 electron microscope that is operated at 75V at a Magnification 60,000 X.

### Electron Microscopy Results

To assess the effect of immunized mouse sera on the assembly of Abeta into fibrils, sera are incubated as described above in the presence or absence of Abeta₄₂ at 37°C for up to 14 days. Aliquots from each reaction mixture are examined at days 1, 3, 7, 10 and 14 for the presence of Abeta₄₂ fibrils by negative stain electron microscopy.

In the absence of sera, or in the presence of non-immunized sera, Abeta₄₂ formed long fibrils (∼7500Å) with a characteristic 50-70 Å diameter. In the presence of sera from IAPP-immunized animals, fewer long Abeta₄₂ fibrils are produced, but the fibrils that did form had the characteristic 50-70 Å diameter. In contrast, the majority of mouse sera from mice immunized with peptide antigens corresponding to SEQ ID NO: 25, 26, 27, 28, 29, 30, 31, 32, 33, 34, 35, 36, 37, 38, 39, 40, 41, 42, 43, 45, 46, 47, 48, 49, 50, and 51 which contain the B-cell epitope Abeta₍₄₋₁₀₎ largely blocked fibril formation, although some sera show little or no effect.

In addition, no difference in the structure of the fibrils is detectable when the fibrils are incubated in the presence of non-immunized mouse sera. Sera from mice that are immunized with IAPP decrease the extent of fibril formation but fibrils that do form are similar to fibrils formed by Abeta₄₂ alone. Finally, sera from mice that are immunized with the peptide antigens corresponding to SEQ ID NO: 25, 26, 27, 28, 29, 30, 31, 32, 33, 34, 35, 36, 37, 38, 39, 40, 41, 42, 43, 45, 46, 47, 48, 49, 50, and 51 inhibit fibrillogenesis to varying extents.

### Sequence Information

| | | | | |
|---|---|---|---|---|
| Number of Residues | | | | |
| 1 | 5 | 10 | 15 | |
| D A E F R H D S G Y | | | | Epitope excis. Maldi/ESI of Aβ42 Protease products presented to AB |
| D A E F R H D S G Y E V H H Q K | | | | trypsin & lys-C-protease epitope excision fragments |
| D A E F R H D S G Y E | | | | *S. Aureus* Glu-C protease |
| DAEFRHDSGY | | | | α-chymotrypsin |
| F R H D S G Y | | | | α-chymotrypsin (Y10) and aminopeptidase M (Dl, A2, E3); |

Abeta 42
1 5 10 15 20
25 30
(APP-672) D A E F R H D S G Y E V H H Q K L V F F A E D V
G S N K G A

31 35 40
I I G L M V G G V V I A-(APP-713)

## Claims

1. A peptide represented by the formula
(A)ₙ - - (Th)ₘ - - (B)ₒ - - Abeta₍₄₋₁₀₎ - - (C)ₚ
wherein each of A, B and C are an amino acid residue or a sequence of amino acid residues;
wherein n, o, and p are independently integers ranging from 0 to about 20;
Th is independently a sequence of amino acid residues that comprises a helper T cell epitope;
when o is equal to 0 then Th is directly connected to the B cell epitope through a peptide bond without any spacer residues;
wherein m is an integer from 1 to about 5, and Abeta₍₄₋₁₀₎ is (SEQ ID NO: 1), wherein said peptide is selected from the group consisting of SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; and SEQ ID NO: 46.

2. A peptide composition comprising a mixture of two or more peptides represented by the formula
(A)ₙ - - (Th)ₘ - - (B)ₒ - - Abeta ₍₄₋₁₀₎ - - (C)ₚ
wherein each of A, B and C are an amino acid residue or a sequence of amino acid residues;
wherein n, o, and p are independently integers ranging from 0 to about 20;
Th is independently a sequence of amino acid residues that comprises a helper T cell epitope;
when o is equal to 0 then Th is directly connected to the B cell epitope through a peptide bond without any spacer residues;
wherein m is an integer from 1 to about 5; and Abeta₍₄₋₁₀₎ is (SEQ ID NO: 1), wherein one of said peptides is selected from the group consisting of SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; and SEQ ID NO: 46.

3. An immunogenic composition for inducing the production of antibodies that specifically bind to an amyloid-beta peptide (SEQ ID NO: 2) comprising:
(a) an antigen, comprising a T-cell epitope that provides an effective amount of T-cell help and a B-cell epitope consisting of peptide Abeta₍₄₋₁₀₎ (SEQ ID NO: 1); wherein said antigen is selected from the group consisting of SEQ ID NO: 25; SEQ ID NO: 26; SEQ ID NO: 27; SEQ ID NO: 28; SEQ ID NO: 29; SEQ ID NO: 30; SEQ ID NO: 31; SEQ ID NO: 32; SEQ ID NO: 33; SEQ ID NO: 34; SEQ ID NO: 35; SEQ ID NO: 36; SEQ ID NO: 37; SEQ ID NO: 38; SEQ ID NO: 39; SEQ ID NO: 40; SEQ ID NO: 41; SEQ ID NO: 42; SEQ ID NO: 43; SEQ ID NO: 44; SEQ ID NO: 45; and SEQ ID NO: 46; and
(b) an adjuvant.

4. The composition of Claim 3, wherein said adjuvant comprises one or more substances selected from the group consisting of aluminium hydroxide, aluminum phosphate, saponin, Quill A, Quill A/ISCOMs, dimethyl dioctadecyl ammomium bromide/arvidine, polyanions, Freunds complete adjuvant, N-acetylmuramyl-L-alanyl-D-isoglutamine, N-acetylmuramyl-L-threonyl-D-isoglutamine, Freund's incomplete adjuvant, and liposomes.

5. The use of an immunogenic composition according to any of Claims 3-4 in the manufacture of a medicament for treating an individual afflicted with Alzheimer's disease.

6. The use of an immunogenic composition according to any of Claims 3-4 in the manufacture of a medicament for reducing the amount of amyloid deposits in the brain of an individual afflicted with Alzheimer's disease.

7. The use of an immunogenic composition according to any of Claims 3-4 in the manufacture of a medicament for disaggregating the amyloid fibrils in the brain of an individual afflicted with Alzheimer's disease.

8. A method for determining if a compound is an inhibitor of amyloid deposition and fibril formation comprising:
(i) contacting the compound with the peptide Abeta₍₄₋₁₀₎ (SEQ ID NO: 1); and
(ii) detecting the binding of the compound with the peptide.

9. The method of Claim 8, further comprising evaluating whether the compound inhibits amyloid fibril formation *in vitro.*

## Patentansprüche

1. Peptid, dargestellt durch die Formel
(A)ₙ - - (Th)ₘ - - (B)ₒ - - Abeta(₄₋₁₀) - - (C)ₚ
wobei jedes von A, B und C ein Aminosäurerest oder eine Sequenz von Aminosäureresten ist;
wobei n, o und p unabhängig voneinander ganze Zahlen im Bereich von 0 bis etwa 20 sind;
Th unabhängig eine Sequenz von Aminosäureresten, welche ein Helfer-T-Zellepitop umfasst, ist;
wobei, wenn o gleich 0 ist, dann Th direkt an das B-Zellepitop durch eine Peptidbindung ohne jedwede Spacer-Reste gebunden ist;
wobei m eine ganze Zahl von 1 bis etwa 5 ist und Abeta₍₄₋₁₀₎ (SEQ ID Nr.: 1) ist, wobei das Peptid aus der Gruppe, bestehend aus SEQ ID Nr.: 25; SEQ ID Nr.: 26; SEQ ID Nr.: 27; SEQ ID Nr.: 28; SEQ ID Nr.: 29; SEQ ID Nr.: 30; SEQ ID Nr.: 31; SEQ ID Nr.: 32; SEQ ID Nr.: 33; SEQ ID Nr.: 34; SEQ ID Nr.: 35; SEQ ID Nr.: 36; SEQ ID Nr.: 37; SEQ ID Nr.: 38; SEQ ID Nr.: 39; SEQ ID Nr.: 40; SEQ ID Nr.: 41; SEQ ID Nr.: 42; SEQ ID Nr.: 43; SEQ ID Nr.: 44; SEQ ID Nr.: 45 und SEQ ID Nr.: 46, ausgewählt ist.

2. Peptidzusammensetzung, umfassend ein Gemisch von zwei oder mehreren Peptiden, dargestellt durch die Formel
(A)ₙ - - (Th)ₘ - - (B)ₒ - - Abeta₍₄₋₁₀₎ - - (C)ₚ
wobei jedes von A, B und C ein Aminosäurerest oder eine Sequenz von Aminosäureresten ist;
wobei n, o und p unabhängig voneinander ganze Zahlen im Bereich von 0 bis etwa 20 sind;
Th unabhängig eine Sequenz von Aminosäureresten, welche ein Helfer-T-Zellepitop umfasst, ist;
wobei, wenn o gleich 0 ist, dann Th direkt an das B-Zellepitop durch eine Peptidbindung ohne jedwede Spacer-Reste gebunden ist;
wobei m eine ganze Zahl von 1 bis etwa 5 ist und Abeta₍₄₋₁₀₎ (SEQ ID Nr.: 1) ist, wobei eines der Peptide aus der Gruppe, bestehend aus SEQ ID Nr.: 25; SEQ ID Nr.: 26; SEQ ID Nr.: 27; SEQ ID Nr.: 28; SEQ ID Nr.: 29; SEQ ID Nr.: 30; SEQ ID Nr.: 31; SEQ ID Nr.: 32; SEQ ID Nr.: 33; SEQ ID Nr.: 34; SEQ ID Nr.: 35; SEQ ID Nr.: 36; SEQ ID Nr.: 37; SEQ ID Nr.: 38; SEQ ID Nr.: 39; SEQ ID Nr.: 40; SEQ ID Nr.: 41; SEQ ID Nr.: 42; SEQ ID Nr.: 43; SEQ ID Nr.: 44; SEQ ID Nr.: 45 und SEQ ID Nr.: 46, ausgewählt ist.

3. Immunogene Zusammensetzung zum Induzieren der Herstellung von Antikörpern, welche spezifisch an ein Amyloid-beta-Peptid (SEQ ID Nr.: 2) binden, umfassend:
(a) ein Antigen, umfassend ein T-Zellepitop, welches eine wirksame Menge von T-Zell-Helfer bereitstellt, und ein B-Zellepitop, bestehend aus Peptid Abeta₍₄₋₁₀₎ (SEQ ID Nr.: 1);
wobei das Antigen aus der Gruppe, bestehend aus SEQ ID Nr.: 25; SEQ ID Nr.: 26; SEQ ID Nr.: 27; SEQ ID Nr.: 28; SEQ ID Nr.: 29; SEQ ID Nr.: 30; SEQ ID Nr.: 31; SEQ ID Nr.: 32; SEQ ID Nr.: 33; SEQ ID Nr.: 34; SEQ ID Nr.: 35; SEQ ID Nr.: 36; SEQ ID Nr.: 37; SEQ ID Nr.: 38; SEQ ID Nr.: 39; SEQ ID Nr.: 40; SEQ ID Nr.: 41; SEQ ID Nr.: 42; SEQ ID Nr.: 43; SEQ ID Nr.: 44; SEQ ID Nr.: 45 und SEQ ID Nr.: 46, ausgewählt ist; und
(b) ein Adjuvans.

4. Zusammensetzung nach Anspruch 3, wobei das Adjuvans eine oder mehrere Substanzen, ausgewählt aus der Gruppe, bestehend aus Aluminiumhydroxid, Aluminiumphosphat, Saponin, Quill A, Quill A/ISCOMs, Dimethyldioctadecylammoniumbromid/Arvidin, Polyanionen, komplettem Freund-Adjuvans, N-Acetylmuramyl-L-alanyl-D-isoglutamin, N-Acetylmuramyl-L-threonyl-D-isoglutamin, nicht komplettem Freund-Adjuvans und Liposomen, umfasst.

5. Verwendung einer immunogenen Zusammensetzung gemäß einem der Ansprüche 3-4 zur Herstellung eines Medikaments zur Behandlung eines Individuums, welches an Alzheimer-Krankheit leidet.

6. Verwendung einer immunogenen Zusammensetzung gemäß einem der Ansprüche 3-4 zur Herstellung eines Medikaments zur Verringerung der Menge von Amyloid-Ablagerungen im Gehirn eines Individuums, welches an Alzheimer-Krankheit leidet.

7. Verwendung einer immunogenen Zusammensetzung gemäß einem der Ansprüche 3-4 zur Herstellung eines Medikaments zum Disaggregieren der Amyloid-Fibrillen im Gehirn eines Individuums, welches an Alzheimer-Krankheit leidet.

8. Verfahren zum Bestimmen, ob eine Verbindung ein Inhibitor von Amyloid-Ablagerungs- und -Fibrillen-Bildung ist, umfassend:
(i) das Inkontaktbringen der Verbindung mit dem Peptid Abeta₍₄₋₁₀₎ (SEQ ID Nr.: 1); und
(ii) das Nachweisen der Bindung der Verbindung mit dem Peptid.

9. Verfahren nach Anspruch 8, ferner umfassend das Bewerten, ob die Verbindung eine Amyloid-Fibrillen-Bildung *in vitro* inhibiert.

## Revendications

1. Peptide représenté par la formule :
(A)ₙ - - (Th)ₘ - - (B)ₒ - - Abeta₍₄₋₁₀₎ - - (C)ₚ
dans laquelle chacun des A, B et C représente un résidu acide aminé ou une séquence de résidus acides aminés ;
dans laquelle n, o, et p sont indépendamment des entiers se situant dans la plage allant de 0 à environ 20 ;
Th représente indépendamment une séquence de résidus acides aminés comprenant un épitope de lymphocyte T auxiliaire ;
lorsque o est égal à 0 alors Th est directement lié à l'épitope de lymphocyte B par le biais d'une liaison peptidique sans aucun résidu espaceur ;
dans laquelle m représente un entier allant de 1 à environ 5, et Abeta₍₄₋₁₀₎ représente la (SEQ ID No. : 1), dans laquelle ledit peptide est choisi dans le groupe comprenant la SEQ ID No. : 25 ; la SEQ ID No. : 26 ; la SEQ ID No. : 27 ; la SEQ ID No. : 28 ; la SEQ ID No. : 29 ; la SEQ ID No. : 30 ; la SEQ ID No. : 31 ; la SEQ ID No. : 32 ; la SEQ ID No. : 33 ; la SEQ ID No. : 34 ; la SEQ ID No. : 35 ; la SEQ ID No. : 36 ; la SEQ ID No. : 37 ; la SEQ ID No. : 38 ; la SEQ ID No. : 39 ; la SEQ ID No. : 40 ; la SEQ ID No. : 41 ; la SEQ ID No. : 42 ; la SEQ ID No. : 43 ; la SEQ ID No. : 44 ; la SEQ ID No. : 45 ; et la SEQ ID No. : 46.

2. Composition peptidique comprenant un mélange de deux peptides ou plus représentés par la formulé :
(A)ₙ - - (Th)ₘ - - (B)ₒ - - Abeta₍₄₋₁₀₎ - - (C)ₚ
dans laquelle chacun des A, B et C représente un résidu acide aminé ou une séquence de résidus acides aminés ;
dans laquelle n, o, et p sont indépendamment des entiers se situant dans la plage allant de 0 à environ 20 ;
Th représente indépendamment une séquence de résidus acides aminés comprenant un épitope de lymphocyte T auxiliaire ;
lorsque o est égal à 0 alors Th est directement lié à l'épitope de lymphocyte B par le biais d'une liaison peptidique sans aucun résidu espaceur ;
dans laquelle m représente un entier allant de 1 à environ 5 ; et Abeta₍₄₋₁₀₎ représente la (SEQ ID No. : 1), dans laquelle un desdits peptides est choisi dans le groupe comprenant la SEQ ID No. : 25 ; la SEQ ID No. : 26 ; la SEQ ID No. : 27 ; la SEQ ID No. : 28 ; la SEQ ID No. : 29 ; la SEQ ID No. : 30 ; la SEQ ID No. : 31 ; la SEQ ID No. : 32 ; la SEQ ID No. : 33 ; la SEQ ID No. : 34 ; la SEQ ID No. : 35 ; la SEQ ID No. : 36 ; la SEQ ID No. : 37 ; la SEQ ID No. : 38 ; la SEQ ID No. : 39 ; la SEQ ID No. : 40 ; la SEQ ID No. : 41 ; la SEQ ID No. : 42 ; la SEQ ID No. : 43 ; la SEQ ID No. : 44 ; la SEQ ID No. : 45 ; et la SEQ ID No. : 46.

3. Composition immunogène destinée à induire la production d'anticorps se liant spécifiquement à un peptide β-amyloïde (SEQ ID No. : 2) comprenant :
(a) un antigène, comprenant un épitope de lymphocyte T fournissant une quantité efficace d'un lymphocyte T auxiliaire et un épitope de lymphocyte B consistant en un peptide Abeta₍₄₋₁₀₎ (SEQ ID No. : 1) ; ledit antigène étant choisi dans le groupe comprenant la SEQ ID No. : 25 ; la SEQ ID No. : 26 ; la SEQ ID No. : 27 ; la SEQ ID No. : 28 ; la SEQ ID No. : 29 ; la SEQ ID No. : 30 ; la SEQ ID No. : 31 ; la SEQ ID No. : 32 ; la SEQ ID No. : 33 ; la SEQ ID No. : 34 ; la SEQ ID No. : 35 ; la SEQ ID No. : 36 ; la SEQ ID No. : 37 ; la SEQ ID No. : 38 ; la SEQ ID No. : 39 ; la SEQ ID No. : 40 ; la SEQ ID No. : 41 ; la SEQ ID No. : 42 ; la SEQ ID No. : 43 ; la SEQ ID No. : 44 ; la SEQ ID No. : 45 ; et la SEQ ID No. : 46 ; et
(b) un adjuvant.

4. Composition selon la revendication 3, dans laquelle ledit adjuvant comprend une ou plusieurs substances choisies dans le groupe comprenant de l'hydroxyde d'aluminium, du phosphate d'aluminium, une saponine, la Quill A, la Quill A / ISCOM, le bromure de diméthyl dioctadécyl ammomium / arvidine, des polyanions, l'adjuvant complet de Freund, la N-acétylmuramyl-L-alanyl-D-isoglutamine, la N-acétylmuramyl-L-thréonyl-D-isoglutamine, l'adjuvant incomplet de Freund, et des liposomes.

5. Utilisation d'une composition immunogène selon l'une quelconque des revendications 3 à 4, dans la fabrication d'un médicament destiné à traiter un individu atteint de la maladie d'Alzheimer.

6. Utilisation d'une composition immunogène selon l'une quelconque des revendications 3 à 4, dans la fabrication d'un médicament destiné à réduire la quantité des dépôts amyloïdes dans le cerveau d'un individu atteint de la maladie d'Alzheimer.

7. Utilisation d'une composition immunogène selon l'une quelconque des revendications 3 à 4, dans la fabrication d'un médicament destiné à désagréger les fibrilles amyloïdes dans le cerveau d'un individu atteint de la maladie d'Alzheimer.

8. Procédé destiné à déterminer si un composé est un inhibiteur de l'accumulation des dépôts amyloïdes et de la formation des fibrilles comprenant les étapes consistant à :
(i) mettre en contact le composé avec le peptide Abeta₍₄₋₁₀₎ (SEQ ID No. : 1) ; et
(ii) détecter la liaison du composé avec le peptide.

9. Procédé selon la revendication 8, comprenant en outre l'étape consistant à évaluer si le composé inhibe la formation des fibrilles amyloïdes *in vitro.*
